# EUROPÄISCHE PATENTSCHRIFT

(11) **EP 1 572 698 B1**
(45) Veröffentlichungstag und Bekanntmachung des Hinweises auf die Patenterteilung: **02.07.2008**
(21) Anmeldenummer: 03813137.1
(22) Anmeldetag: 17.12.2003
(51) Int. Cl.: C07D 495/14, A61K 31/519, A61P 25/00

(54) **3-SUBSTITUIERTE 3,4-DIHYDRO-THIENO¬2,3-D PYRIMIDIN-4-ONE-DERIVATE, IHRE HERSTELLUNG UND VERWENDUNG**
3-SUBSTITUTED 3,4-DIHYDRO-THIENO¬2,3-D PYRIMIDINE-4-ONE-DERIVATIVES, PRODUCTION AND USE THEREOF
DERIVES DE 3,4-DIHYDRO-THIENO¬2,3-D PYRIMIDIN-4-ONE 3-SUBSTITUES, LEUR PREPARATION ET LEUR UTILISATION

(30) Priorität: 18.12.2002 DE 10259382
(43) Veröffentlichungstag der Anmeldung: 14.09.2005
(73) Patentinhaber: Abbott GmbH & Co. KG, 65205 Wiesbaden (DE)
(72) Erfinder: BAKKER, Margaretha, 64342 Seeheim - Jungenheim (DE); HORNBERGER, Wilfried, 67434 Neustadt (DE); KLING, Andreas, 68239 Mannheim (DE); LANGE, Udo, 13639 BERLIN (DE); MACK, Helmut, 67067 Ludwigshafen (DE); MOELLER, Achim, 67269 Grünstadt (DE); MUELLER, Reinhold, 67105 Schifferstadt (DE); SCHELLHAAS, Kurt, 67067 Ludwigshafen (DE); SCHMIDT, Martin, 64625 Bensheim (DE); STEINER, Gerd, 67281 Kirchheim (DE); WICKE, Karsten, 67122 Altrip (DE)
(74) Vertreter: Reitstötter - Kinzebach
(86) Internationale Anmeldenummer: PCT/EP2003/014423
(87) Internationale Veröffentlichungsnummer: WO 2004/055024

(56) Entgegenhaltungen:
- EP-A- 0 196 132
- WO-A-98/11110
- WO-A-98/56793
- WO-A-99/07711

## Beschreibung

Die Erfindung betrifft 3-substituierte 3,4-Dihydro-thieno[2,3-d]pyrimidin-4-on-Derivate, ihre Herstellung und ihre Verwendung insbesondere zu therapeutischen Zwecken, z.B. bei der Behandlung von Depressionen.

ZNS-Erkrankungen betreffen heutzutage große Bevölkerungsteile. Insbesondere sind etwa 20 % aller Frauen und 12 % aller Männer in ihrem Leben von psychiatrischen Erkrankungen wie Gemütsstörungen, z.B. Depressionen betroffen.

Derzeit werden eine Vielzahl von Strategien zur Behandlung psychiatrischer Erkrankungen diskutiert und teilweise auch angewendet. Beispielsweise werden selektive Dopamin D4/5-HT₂-Rezeptor-Antagonisten, wie Risperidon (vgl. auch EP 0 196 132) und Belaperidon, 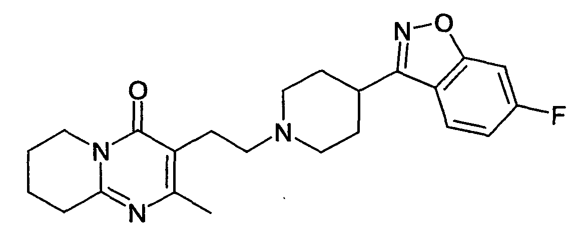 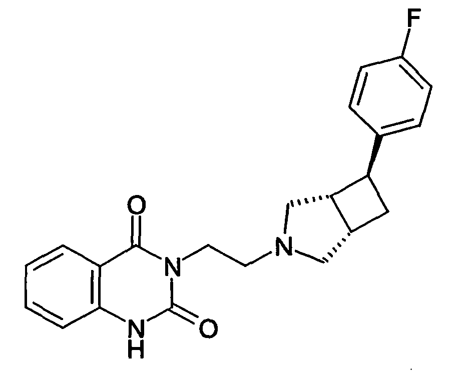 für die Behandlung von Psychosen vorgeschlagen.

Einem anderen Ansatz zufolge versucht man, einen als das antidepressive Wirkprinzip geltenden Anstieg der Serotoninkonzentration im synaptischen Spalt dadurch zu bewirken, dass man einerseits die aktive Wiederaufnahme (Reuptake) von Serotonin und/oder präsynaptische Serotonin-Autorezeptoren medikamentös blockiert.

Beispielsweise entfalten die klassischen Antidepressiva und auch die neueren selektiven Serotonin-Reuptakehemmer (SSRIs), wie z.B. Paroxetine oder Fluoxetine, ihre antidepressive Wirkung unter anderem durch die Inhibierung der aktiven Wiederaufnahme des Transmitters in die präsynaptischen Nervenendigungen. Leider tritt dabei die antidepressive Wirkung erst nach einer Behandlungsdauer von mindestens 3 Wochen ein. Zudem sind etwa 30 % der Patienten therapieresistent.

Durch die Blockade präsynaptischer Serotonin-Autorezeptoren soll die negative Rückkopplung auf die Serotoninfreisetzung aufgehoben und damit die aktuelle Transmitterkonzeritration erhöht werden.

Nach bisherigem Wissen handelt es sich bei dem präsynaptischen Serotonin-Autorezeptor um den 5-HT_{1B/D}-Subtyp. Es sei bemerkt, dass die beiden den humanen 5-HT_{1B/D}-Subtyp kodierenden Gene zunächst mit 5-HT_{1Dα} und 5-HT_{1Dβ} bezeichnet worden waren und anschließend die Bezeichnungen 5-HT_{1B} und 5-HT_{1D} erhielten. Die selektive Blockade durch 5-HT_{1B/D}-Antagonisten sollte demnach die Serotonin-Freisetzung im Gehirn erhöhen. Jedoch wird die Serotonin-Freisetzung im Cortex nach systemischer Gabe des selektiven 5-HT_{1B}-Antagonisten GR 127 935 überraschenderweise vermindert. Eine Erklärung könnte die Stimulierung von somatodendritischen 5-HT_{1A}-Rezeptoren in der Raphe Region durch das freigesetzte Serotonin sein, wodurch die Feuerrate serotonerger Neuronen und damit die Serotonin-Ausschüttung gehemmt wird.

Einer weiteren Strategie zufolge versucht man daher, die autoinhibitorischen Effekte in serotonergen Ursprungsgebieten zu umgehen, indem man sowohl die präsynaptischen 5-HT_{1B/D}-Rezeptoren als auch den 5-HT_{1A}-Rezeptor blockiert, um die terminale Serotonin-Freisetzung anzuheben bzw. das neuronale Feuern zu verstärken (Starkey and Skingle, Neuropharmacology (1994) 33 (3-4) 393; vgl. auch WO 95/31988).

Dieser Strategie folgend werden bestimmte 3-substituierte 3,4-Dihydro-thieno[2,3-d]pyrimidin-Derivate (WO 98/11110; WO 98/56792; WO 98/56793; WO 99/07711) mit einer hohen Affinität zu den Serotoninrezeptoren 5-HT_{1B/D} und 5-HT_{1A} als Wirkstoffe, beispielsweise zur Behandlung von Depressionen, vorgeschlagen. Darüber hinaus werden neurodegenerative und neuropsychiatrische Erkrankungen in WO 00/41696 und die zerebrale Ischämie in WO 00/41695 als weitere therapeutische Verwendungen dieser Derivate angegeben.

Überraschenderweise stellte man nun fest, dass weitere 3-substituierte 3,4-Dihydrothieno[2,3-d]pyrimidin-Derivate besonders wertvolle pharmakologische Eigenschaften aufweisen und daher in besonderer Weise für die Therapie geeignet erscheinen.

Gegenstand der vorliegenden Erfindung sind daher Verbindung der Formel (I) 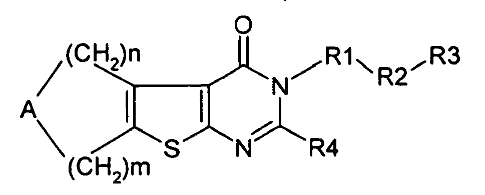 worin
- A: für O, S, SO, NR5 oder CH₂ steht;
- R5: für H, C₁₋₅-Alkyl, Aryl, Aralkyl, Acyl oder Alkoxycarbonyl steht;
- R4: für H oder Methyl steht;
- n: 1 oder 2 bedeutet;
- m: 1 oder 2 bedeutet;
- R1: für C₁₋₈-Alkylen steht;
- R2: für eine Gruppe der Formel 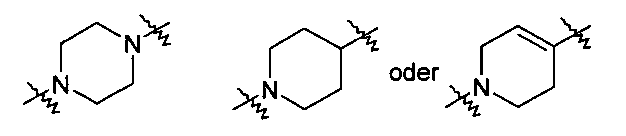
steht;
- R3: für 5-gliedriges Heteroaryl steht, das mit einem Aryl- oder Heteroarylrest kondensiert sein kann, wobei das Heteroaryl und gegebenenfalls der kondensierte Aryl- oder Heteroarylrest 1, 2 oder 3 unabhängig voneinander unter C₁₋₅-Alkyl, C₁₋₅-Alkoxy, C₁₋₅-Alkylthio, Halogen, CN, Halogen-C₁₋₅-alkyl, Halogen-C₁₋₅-alkoxy, Hydroxy, -NH₂, -N(R6)₂, -NH(R6), Aryl, Aryloxy, Aralkyl, Aralkyloxy und Heteroaryl ausgewählte Substituenten tragen können, wobei die Substituenten Aryl, Aryloxy, Aralkyl, Aralkyloxy und Heteroaryl 1, 2 oder 3 unabhängig voneinander unter C₁₋₅-Alkyl, C₁₋₅-Alkoxy, C₁₋₅-Alkylthio, Halogen, CN, Halogen-C₁₋₅-alkyl, Halogen-C₁₋₅-alkoxy, Hydroxy, -NH₂, -N(R6)₂ und -NH(R6) ausgewählte Substituenten tragen können; und die Reste
- R6: unabhängig voneinander für C₁₋₅-Alkyl stehen,
sowie physiologisch verträgliche Salze davon.

Bevorzugte Ausführungsformen dieses Gegenstandes sind in den anliegenden Ansprüchen beschrieben.

Zu den physiologisch verträglichen Salzen gehören im vorliegenden Fall vornehmlich Säureadditionssalze, wozu insbesondere Salze der erfindungsgemäßen Verbindungen mit anorganischen Säuren, wie Salzsäure, Schwefelsäure, Salpetersäure oder Phosphorsäure, oder organischen Säuren, insbesondere Carbonsäuren, z.B. Essigsäure, Weinsäure, Milchsäure, Citronensäure, Äpfelsäure, Mandelsäure, Ascorbinsäure, Maleinsäure, Fumarsäure, Gluconsäure oder Sulfonsäuren, z. B. Methansulfonsäure, Benzolsulfonsäure und Toluolsulfonsäure, und dergleichen zählen.

Soweit die erfindungsgemäßen Verbindungen Asymmetriezentren aufweisen, sind Racemate sowie optische Isomere als Gemische oder in Reinform (Enantiomere, Diastereomere) umfasst. Für therapeutische Zwecke kann man Enantiomeren-Gemische verwenden. Diastereomere werden wegen unterschiedlicher physikochemischer Eigenschaften vorzugsweise als im Wesentlichen reine Diastereomere verwendet.

Der Begriff "Alkyl, Alkoxy etc." umfasst geradkettige oder verzweigte Alkylgruppen, wie -CH₃, -C₂H₅, n-Propyl, -CH(CH₃)₂, n-Butyl, -CH(CH₃)-C₂H₅, isobutyl, -C(CH₃)₃, n-Pentyl oder n-Hexyl, insbesondere CH₃, C₂H₅ oder CH(CH₃)₂, vorzugsweise mit - soweit nicht anderes angegeben ist - 1 bis 8, insbesondere 1 bis 6 und besonders bevorzugt 1 bis 5 Kohlenstoffatomen; als Substituent des Rests R3 umfaßt "Alkyl, Alkoxy etc." vorzugsweise 1 bis 3 Kohlenstoffatome. Alkylthio steht vorzugsweise für -SCH₃.

Der Begriff "Halogenalkyl" meint Alkyl, das partiell oder vollständig durch Fluor, Chlor, Brom und/oder Iod substituiert ist, also z.B. CH₂F, CHF₂, CF₃, CH₂Cl, 2-Fluorethyl, 2-Chlorethyl oder 2,2,2-Trifluorethyl; als Substituent des Rests R3 bedeutet Halogenalkyl vorzugsweise CHF₂ und vor allem CF₃.

Der Begriff "Halogenalkoxy" meint Alkoxy, das partiell oder vollständig durch Fluor, Chlor, Brom und/oder Iod substituiert ist, also z.B die den vorstehend aufgezählten Halogenalkylresten entsprechenden Halogenalkoxyreste; als Substituent des Rests R3 bedeutet Halogenalkoxy vorzugsweise OCHF₂ und vor allem OCF₃.

Acyl meint -COR, worin R für Alkyl, Aryl oder Aralkyl stehen kann. Demnach steht Acyl insbesondere für C₁₋₅-Alkyl-CO-, Aryl-CO-, Arylmethyl-CO- und Arylethyl-CO-, z.B. Acetoxy und Benzoyl.

Alkoxycarbonyl meint -COOAlkyl mit vorzugsweise 1 bis 5 Kohlenstoffatomen im Alkylteil, also C₁₋₅-Alkyl-O-CO-, wie -CO-OCH₃, -CO-OC₂H₅, -CO-OCH₂-C₂H₅, -CO-OCH(CH₃)₂, n-Butoxycarbonyl, -CO-OCH(CH₃)-C₂H₅, -CO-OCH₂-CH(CH₃)₂, -CO-OC(CH₃)₃, insbesondere -CO-OCH₃, -CO-OC₂H₅, -CO-OCH(CH₃)₂ oder -CO-OCH₂-CH(CH₃)₂.

Aryl meint insbesondere einen Aromaten mit einer 5- oder 6-gliedrigen Ringstruktur, der mit einem Arylrest kondensiert sein kann und steht vorzugsweise für Naphthyl und insbesondere für Phenyl.

Heteroaryl meint insbesondere einen 1, 2, 3 oder 4 unabhängig unter O, N und S ausgewählte Heteroatome umfassenden Aromaten mit einer die Hetero- und Kohlenstoffatome umfassenden 5- oder 6-gliedrigen Ringstruktur, der mit einem Aryl- oder Heteroarylrest kondensiert sein kann. Zu Heteroaryl mit 6-gliedriger Ringstruktur gehören stickstoffhaltige Reste, wie Pyridyl, Pyrimidyl, Pyridazinyl, Pyrazinyl, Chinolinyl und Isochinolinyl.

Der Begriff "5-gliedriges Heteroaryl" meint insbesondere einen 1, 2, 3 oder 4 unabhängig unter O, N und S ausgewählte Heteroatome umfassenden Aromaten mit einer die Hetero- und Kohlenstoffatome umfassenden 5-gliedrigen Ringstruktur. Hierzu gehören stickstoffhaltige Reste, wie Pyrrolyl, Imidazolyl, Pyrazolyl, 1,2,4-Triazolyl, Tetrazolyl; sauerstoffhaltige Reste, wie Furanyl; schwefelhaltige Reste, wie Thienyl; und Reste mit mindestens zwei verschiedenen Heteroatomen, wie Thiazolyl, Isothiazolyl, 1,3,4-Thiadiazolyl, 1,2,4-Thiadiazolyl, 1,2,5-Thiadiazolyl, Isoxazolyl, Oxazolyl.

Bei 5-gliedrigem Heteroaryl, das mit einem Aryl- oder Heteroarylrest kondensiert ist, handelt es sich insbesondere um vorstehend erläutertes Heteroaryl, das mit 6-gliedrigem Aryl oder Heteroaryl kondensiert ist. Insbesondere sind in diesem Zusammenhang 5-gliedriges Heteroaryl, das mit einem Phenylrest kondensiert ist, d.h. benzoanelliertes Heteroaryl, wie Indolyl, Benzofuranyl, Benzothienyl, Indazolyl, Benzimidazolyl, Benztriazolyl, Benzoxazolyl, Benzisoxazolyl, Benzthiazolyl, Benzisothiazolyl, und 5-gliedriges Heteroaryl, das mit einem Pyridylrest kondensiert ist, wie Pyrrolopyridinyl und Pyridisoxazolyl, zu nennen.

Aryloxy meint über Sauerstoff gebundenes Aryl.

Aralkyl meint über Alkylen gebundenes Aryl, wobei Alkylen hier vorzugsweise 1 bis 3 Kohlenstoffatome aufweist und steht vorzugsweise für Benzyl.

Aralkyloxy meint über Sauerstoff gebundenes Aralkyl, insbesondere Aryl-CH₂-O-, z.B. Benzyloxy.

Der Begriff "Halogen" umfasst ein Fluor-, Chlor-, Brom- oder Iodatom und insbesondere ein Fluor-, Chlor- oder Bromatom. Bevorzugt sind in der Regel Fluor- und Chloratome, gegebenenfalls auch Bromatome.

Sind aromatische Reste erfindungsgemäß substituiert, so sind die Substituenten vorzugsweise auszuwählen unter C₁₋₅-Alkyl, C₁₋₅-Alkoxy, C₁₋₅-Alkylthio, Halogen, CN, Halogen-C₁₋₅-alkyl, Halogen-C₁₋₅-alkoxy, Hydroxy, -NH₂, -N(R6)₂, -NH(R6), Aryl, Aryloxy und Aralkyloxy.

Der Begriff "Alkylen" umfasst geradkettige oder verzweigte, zweiwertige Alkylengruppen, vorzugsweise mit - soweit nicht anderes angegeben ist - 1 bis 8, insbesondere 1 bis 6 und besonders bevorzugt 1 bis 4 Kohlenstoffatomen. Insbesondere sind Alkylenreste wie Methylen, Eth-1,2-ylen, Prop-1,2-ylen, Prop-1,3-ylen, But-1,2-ylen, But-1,3-ylen, But-2,3-ylen, But-1,4-ylen, 2-Methylprop-1,3-ylen, Pent-1,2-ylen, Pent-1,3-yfen, Pent-1,4-ylen, Pent-1,5-ylen, Pent-2,3-ylen, Pent-2,4-ylen, 1-Methylbut-1,4-ylen, 2-Methylbut-1,4-ylen, 2-Methylbut-1,3-ylen, 2-Ethylprop-1,3-ylen, Hex-3,4-ylen, 3-Methylpent-2,4-ylen, Hept-3,5-ylen, 2-Ethylpent-1,3-ylen, 3-Ethylhept-3,5-ylen, etc. zu nennen.

Die erfindungsgemäßen Verbindungen gehören zur Klasse der 3-substituierten 3,4-Dihydro-thieno[2,3-d]pyrimidin-4-on-Derivate. In 5,6-Position weisen diese Verbindungen einen zykloaliphatischen oder heterozyklischen Rest auf, der über die in 5- bzw. 6-Position ansetzenden Alkylenreste (CH₂)ₙ bzw. (CH₂)ₘ sowie den zweiwertigen Rest A gebildet wird.

Vorzugsweise weisen die erfindungsgemäßen Verbindungen in 5,6-Position des Thieno[2,3-d]pyrimidin-4-on-Gerüsts einen Heterozyklus auf. Vorteilhafterweise steht der Rest A für O, S und insbesondere für NR5.

Steht A für NR5, so bedeutet R5 vorzugsweise Wasserstoff oder C₁₋₅-Alkyl, vorteilhafterweise Wasserstoff oder Methyl.

Der zyklische oder heterozyklische, aliphatische, in 5,6-Position des Thieno[2,3-d]pyrimidin-4-on-Gerüsts gebundene Rest bildet vorzugsweise einen 5- und insbesondere 6-gliedrigen Zyklus. Die Summe aus n + m bedeutet daher vorzugsweise 2 und insbesondere 3.

Gemäß einer Ausführungsform betrifft die vorliegende Erfindung 3-substituierte 5,6,7,8-Tetrahydro-pyridothieno[2,3-d]pyrimidin-4(3H)-on-Derivate, nämlich Verbindungen der Formel (I), worin A für NR5 steht und die Summe aus n + m = 3 ist. Hierzu gehören insbesondere 3-substituierte 5,6,7,8-Tetrahydro-pyrido[4',3':4,5]thieno[2,3-d]pyrimidin-4(3H)-on-Derivate (n = 2; m = 1) und 3-substituierte 5,6,7,8-Tetrahydropyrido[3',4':4,5]thieno[2,3-d]pyrimidin-4(3H)-on-Derivate (n = 1; m = 2), wobei erstere bevorzugt sind.

Gemäß einer weiteren Ausführungsform betrifft die vorliegende Erfindung 3-substituierte 3,5,6,8-Tetrahydro-4H-pyrano[4',3':4,5]thieno[2,3-d]pyrimidin-4-on-, 3-substituierte 3,5,6,8-Tetrahydro-4H-thiopyrano[4',3':4,5]thieno[2,3-d]pyrimidin-4-on- und 3-substituierte 5,6,7,8-Tetrahydro[1]benzothieno[2,3-d]pyrimidin-4(3H)-on-Derivate, nämlich Verbindungen der Formel (I), worin A für Sauerstoff, Schwefel bzw. Methylen steht, und n = 2 und m = 1 sind.

Gemäß einer weiteren Ausführungsform betrifft die vorliegende Erfindung 3-substituierte 3,5,6,7-Tetrahydro-4H-pyrrolo[3',4':4,5]thieno[2,3-d]pyrimidin-4-on-, 3-substituierte 5,7-Dihydrofuro[3',4':4,5]thieno[2,3-d]pyrimidin-4(3H)-on-, 3-substituierte 5,7-Dihydrothieno[3',4':4,5]thieno[2,3-d]pyrimidin-4(3H)-on- und 3-substituierte 3,5,6,7-Tetrahydro-4H-cyclopenta[4,5]thieno[2,3-d]pyrimidin-4-on-Derivate, nämlich Verbindungen der Formel (I), worin A für NR5, Sauerstoff, Schwefel bzw. Methylen steht, und n = 1 und m = 1 sind, wobei Verbindungen mit A = NR5 und Methylen bevorzugt sind.

In 2-Position sind die erfindungsgemäßen Verbindungen vorzugsweise nicht substituiert, d.h. R4 ist Wasserstoff.

Der Substituent in 3-Position des Thieno[2,3-d]pyrimidin-4-on-Gerüsts setzt sich aus einer einen gewissen Abstand zum Gerüst schaffenden zweiwertigen Gruppierung R1, einem 5-, 6- oder 7-gliedrigen aliphatischen, monozyklischen, 1 oder 2 Stickstoffatome enthaltenden Heterozyklus R2 und einer terminalen heteroaromatischen Gruppierung R3 zusammen.

Dementsprechend steht R1 für Alkylen, das geradkettig oder verzweigt sein kann und dessen Hauptkette vorzugsweise eine Länge von 2 oder 3 und insbesondere von 2 Kohlenstoffatomen aufweist. Ist das Alkylen verzweigt, weist die Hauptkette vorzugsweise einen unter Methyl und Ethyl ausgewählten Substituenten auf. Zu bevorzugten Alkylenresten R1 gehören daher insbesondere die geradkettigen Reste Eth-1,2-ylen und Prop-1,3-ylen sowie die verzweigten Reste Prop-1,2-ylen, 2-Methyl-prop-1,3-ylen, But-1,2-ylen und But-1,3-ylen.

Der stickstoffhaltige Heterozyklus R2 kann gesättigt oder ungesättigt sein. Ungesättigte Heterozyklen weisen 1 Doppelbindung auf. Üblicherweise ist der Heterozyklus mit dem Rest R1 über ein Stickstoffatom und mit dem Rest R3 über ein Stickstoff- oder ein Kohlenstoffatom verbunden. Weist der Heterozyklus eine Doppelbindung auf, so sitzt diese bevorzugt an dem an R3 gebundenen Kohlenstoffatom. Bei 6-gliedrigen Heterozyklen erfolgt die Verknüpfung der Reste R1 und R3 bevorzugt über die Positionen 1 und 4.

Die vorliegende Erfindung betrifft Verbindungen der Formel (I), worin R2 für einen 6-gliedrigen Heterozyklus steht, der 1 oder 2 Stickstoffatome aufweist und eine Doppelbindung aufweisen kann, nämlich eine Gruppe der Formel 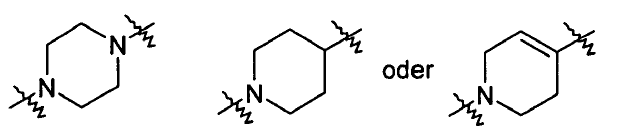 (Piperazin-1,4-yl, Piperidin-1,4-yl oder 1,2,3,6-Tetrahydro-pyridin-1,4-yl), wobei die 1-Position bevorzugt an den Rest R1 und die 4-Position bevorzugt an den Rest R3 bindet.

Es ist zu beachten, dass je nach Art des heteroaromatischen Restes R3 sich für den heterozyklischen Rest R2 vorteilhafte Ausführungsformen ergeben können. Vorzugsweise ist R2 so zu wählen, dass die Gruppierung R2-R3 kein Enamin bildet.

Die erfindungsgemäßen Verbindungen sind dadurch gekennzeichnet, dass der Rest R2 an einen 5-gliedrigen heteroaromatischen Rest (Heteroaryl) gebunden ist. Zudem können diese 5-gliedrigen heteroaromatischen Ringe weitere kondensierte (anellierte) Ringe aufweisen; hierbei teilen sich der jeweilige 5-gliedrige aromatische Ring und ein weiterer aromatischer (Aryl) oder heteroaromatischer (Heteroaryl) Ring zwei benachbarte Ringatome.

Gemäß einer besonderen Ausführungsform betrifft die vorliegende Erfindung Verbindungen der Formel (I), worin R3 für eine Gruppe der Formel (VIII) 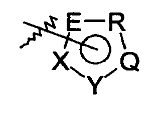 steht, worin
- E: für N oder C steht;
- X: für N, S, C oder CR10 steht;
- Y: für N, NR11, O, S oder CR11 steht;
- Q: für N oder CR9 steht;
- R: für N, NR8 oder CR9 steht;
- R8, R9, R10, R11: unabhängig voneinander für Wasserstoff, C₁₋₅-Alkyl, C₁₋₅-Alkoxy, C₁₋₅-Alkylthio, Halogen, CN, Halogen-C₁₋₅-Alkyl, Halogen-C₁₋₅-Alkoxy, Hydroxy, -NH₂, -N(R6)₂, -NH(R6), Aryl, Aryloxy, Aralkyl, Aralkyloxy und Heteroaryl stehen, wobei die Substituenten Aryl, Aryloxy, Aralkyl, Aralkyloxy oder Heteroaryl wiederum substituiert sein können, oder R8 und R9 zusammen genommen mit den Stickstoff- oder Kohlenstoffatomen, an die sie gebunden sind, einen gegebenenfalls substituierten 5- oder 6-gliedrigen Aryl- oder Heteroarylrest bilden; und die Reste
- R6: unabhängig voneinander obige Bedeutung besitzen.

Von den vorstehend offenbarten Verbindungen der Formel (I) sind insbesondere diejenigen bevorzugt, in denen R3 für eine Gruppe der Formel (VIIIa) 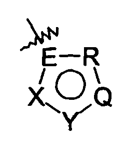 steht, worin E, X, Y, Q, R, R8, R9, R10, R11 und R6 obige Bedeutungen besitzen.

Gemäß einer besonderen Ausführungsform betrifft die vorliegende Erfindung Verbindungen der Formel (I), worin R3 für eine Gruppe der Formel (VIIIa) steht, und E Kohlenstoff, X Stickstoff und Y Sauerstoff sind. Es ist zudem von Vorteil, wenn diese 5-gliedrigen heteroaromatischen Reste anelliert sind, wobei sich Benzol und Pyridin insbesondere als Anelland anbieten.

Gemäß der zuvor beschriebenen Ausführungsform betrifft die vorliegende Erfindung daher besonders vorteilhafte Verbindungen der Formel (I), worin R3 für eine anellierte Gruppe der Formel (IX) 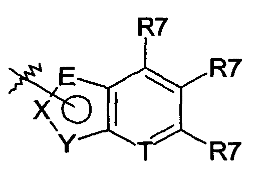 steht, worin
- E: für N oder C steht;
- X: für N, S, C oder CH steht;
- Y: für N, NR11, O, S oder CR11 steht;
- T: für CR7 oder N steht;
- R11: obige Bedeutungen besitzt und vorzugsweise für H oder C₁₋₅-Alkyl steht; und
- die Reste R7: unabhängig voneinander für H, C₁₋₅-Alkyl, C₁₋₅-Alkoxy, C₁₋₅-Alkylthio, Halogen, CN, Halogen-C₁₋₅-Alkyl, Halogen-C₁₋₅-Alkoxy, Hydroxy, -NH₂, -N(R6)₂ oder -NH(R6) stehen.

Von den vorstehend offenbarten besonders vorteilhaften Verbindungen der Formel (I) sind insbesondere diejenigen bevorzugt, in denen R3 für eine Gruppe der Formel (IXa) 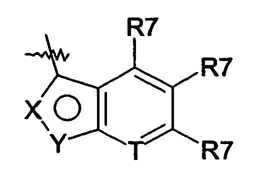 steht, worin X, Y, T, die Reste R7, R11 und R7 obige Bedeutungen besitzen.

Somit betrifft die Erfindung gemäß dieser Ausführungsform insbesondere Verbindungen der Formel (I), worin R3 für gegebenenfalls substituiertes 1 H-Indol-3-yl, 1 H-Pyrrolo[2,3-b]pyridin-3-yl, 1-Benzofuran-3-yl, 1-Benzothien-3-yl, 1H-Indazol-3-yl, 1H-Benzimidazol-1-yl, 1H-Benzimidazol-2-yl, 1H-Benzotriazol-1-yl, 1,3-Benzoxazol-2-yl, 1,2-Benzisoxazol-3-yl, 1,3-Benzothiazol-2-yl und 1,2-Benzisothiazol-3-yl steht, wobei 1,2-Benzisoxazol-3-yl besonders bevorzugt ist.

Von den nicht annelierten 5-gliedrigen heteroaromatischen Gruppen sind insbesondere 1H-Pyrazol-3-yl, 1H-Tetrazol-5-yl, 1,3-Thiazol-2-yl und 1,2,4-Thiadiazol-5-yl zu nennen. Diese können 1, 2 oder 3 unabhängig voneinander unter C₁₋₅-Alkyl, C₁₋₅-Alkoxy, C₁₋₅-Alkylthio, Halogen, CN, Halogen-C₁₋₅-Alkyl, Halogen-C₁₋₅-Alkoxy, Hydroxy, -NH₂, -N(R6)₂ oder -NH(R6), Aryl, Aryloxy, Aralkyl, Aralkyloxy und Heteroaryl ausgewählte Substituenten tragen, wobei die Substituenten Aryl, Aryloxy, Aralkyl, Aralkyloxy und Heteroaryl wiederum 1, 2 oder 3 unabhängig voneinander unter C₁₋₅-Alkyl, C₁₋₅-Alkoxy, Halogen, CN, SCH₃, Trifluormethyl, Hydroxy, -N(C₁₋₅-Alkyl)₂, -NH(C₁₋₅-Alkyl) oder -NH₂ ausgewählte Substituenten tragen können.

Einem weiteren Aspekt der Erfindung zufolge sind die zuvor in Zusammenhang mit dem Rest R3 beschriebenen heteroaromatischen Gruppen einfach substituiert. Dementsprechend steht R3 vorzugsweise für einen Rest der Formel 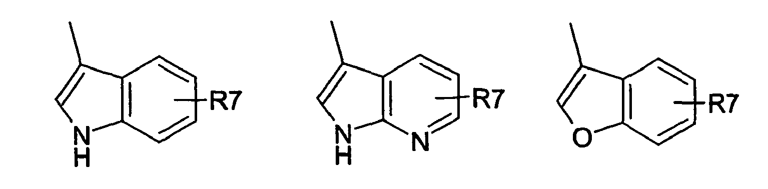 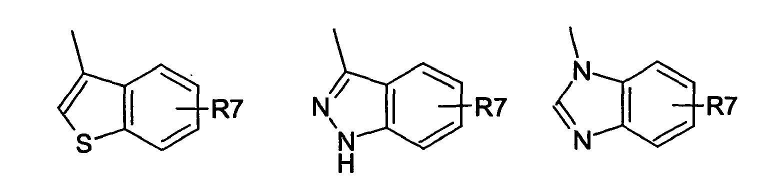 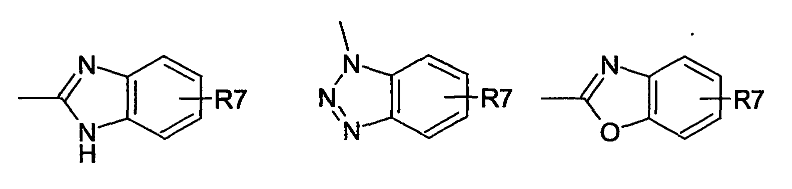 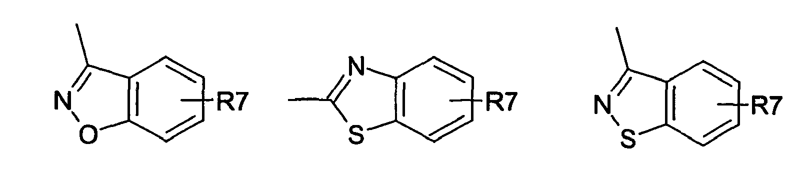 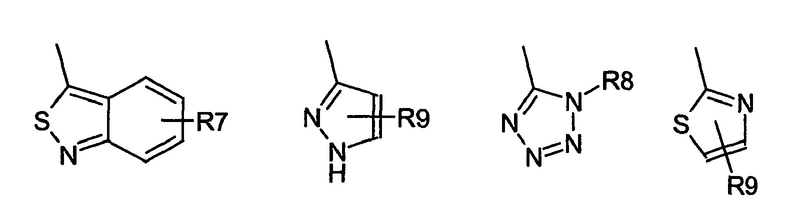 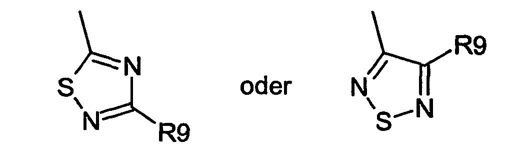 worin
- R7: für H, C₁₋₅-Alkyl, C₁₋₅-Alkoxy, C₁₋₅-Alkylthio, Halogen, CN, Halogen-C₁₋₅-Alkyl, Halogen-C₁₋₅-Alkoxy, Hydroxy, -NH₂, -N(R6)₂ oder -NH(R6) steht;
- R8: für H, C₁₋₅-Alkyl, Aryl, Aralkyl und Heteroaryl steht, wobei Aryl, Aralkyl und Heteroaryl wiederum in der oben beschriebenen Weise substituiert sein können; und
- R9: für H, C₁₋₅-Alkyl, C₁₋₅-Alkoxy, C₁₋₅-Alkylthio, Halogen, CN, Halogen-C₁₋₅-Alkyl, Halogen-C₁₋₅-Alkoxy, Hydroxy, -NH₂, -N(R6)₂, -NH(R6), Aryl, Aryloxy, Aralkyl, Aralkyloxy oder Heteroaryl steht, wobei Aryl, Aryloxy, Aralkyl, Aralkyloxy und Heteroaryl in der oben beschriebenen Weise wiederum substituiert sein können.

Gemäß einer besonders bevorzugten Ausführungsform betrifft die vorliegende Erfindung Verbindungen der Formel (I), worin R3 für eine Gruppe der Formel 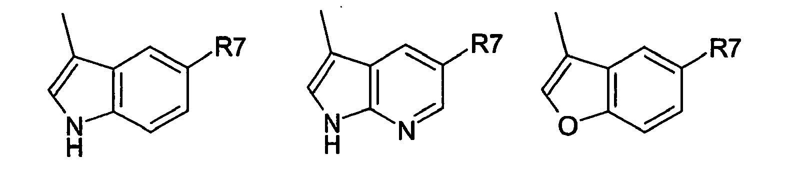 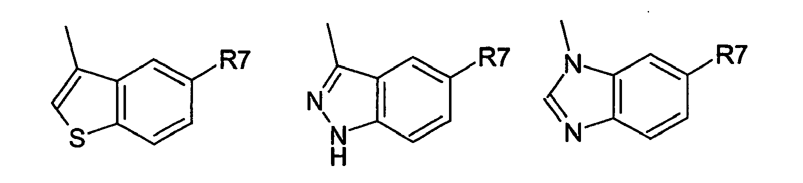 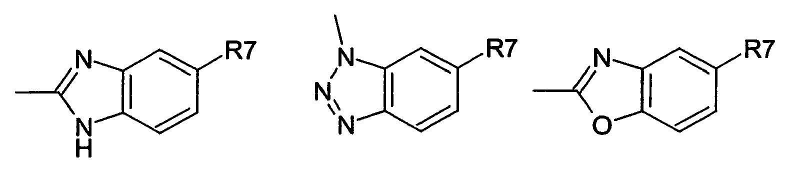 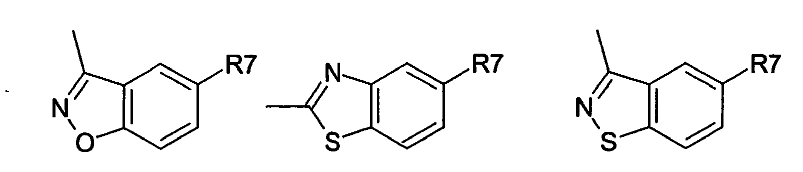 oder 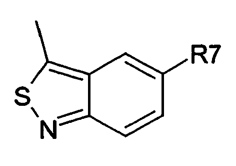 steht, wobei R7 wie oben definiert ist. Ganz besonders bevorzugt sind hiervon der Benzisoxazol-3-yl- und der 1 H-Pyrrolo[2,3-b]pyridin-3-yl-Rest.

Gemäß einer weiteren besonders bevorzugten Ausführungsform betrifft die vorliegende Erfindung Verbindungen der Formel (I), worin R3 für eine Gruppe der Formel 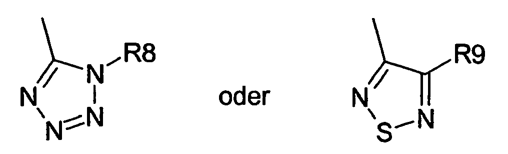 steht, wobei R8 und R9 wie oben definiert sind.

In den erfindungsgemäßen Verbindungen der zuvor erläuterten Ausführungsformen steht R7 als Substituent der anellierten heteroaromatischen Reste insbesondere für C₁₋₅-Alkyl, vorzugsweise Methyl, Halogen, vorzugsweise Chlor, oder Halogen-C₁₋₅-Alkyl, vorzugsweise Trifluormethyl; R8 als Substituent nicht anellierter heteroaromatischer Reste insbesondere für C₁₋₅-Alkyl, vorzugsweise Methyl, Ethyl oder Isopropyl, oder Aryl, vorzugsweise Phenyl, das substituiert sein kann; und R9 als Substituent nicht anellierter heteroaromatischer Reste insbesondere für C₁₋₅-Alkoxy, vorzugsweise Methoxy, Ethoxy oder Isopropoxy, Aryl, vorzugsweise Phenyl, das substituiert sein kann, z.B. mit Chlor, oder Heteroaryl, z.B. 2-Thienyl.

Die erfindungsgemäßen Verbindungen sind in an sich bekannter Weise herstellbar. Insbesondere wird auf die in WO 98/11110; WO 98/56792; WO 98/56793 und WO 00/41695 offenbarten Verfahren zur Herstellung der darin beschriebenen Thieno[2,3-d]pyrimidin-4-on-Derivate Bezug genommen. Dementsprechend kann man verschiedene Vorgehensweisen wählen, die sich grundsätzlich darin unterscheiden, dass man den Substituenten in 3-Position des Thieno[2,3-d]pyrimidin-4-on-Gerüsts als einen bereits die Reste R1, R2 und R3 umfassenden Baustein einführt, oder zunächst einen lediglich den Rest R1 bzw. die Reste R1 und R2 umfassenden Baustein einführt und anschließend mit weiteren, die Reste R2 und R3 bzw. R3 umfassenden Bausteinen in geeigneter Weise umsetzt.

Gegenstand der vorliegenden Erfindung ist daher auch ein Verfahren zur Herstellung erfindungsgemäßer Verbindungen der Formel (I) durch Umsetzung einer Verbindung der Formel (II) 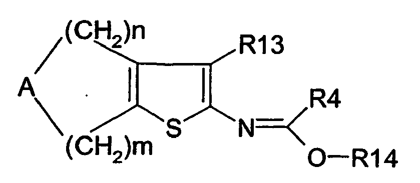 worin A, n, m und R4 eine der oben angegebenen Bedeutung besitzen; R13 für CN oder C₁₋₃-Alkyl-O-CO- steht und R14 für C₁₋₃-Alkyl steht,
mit einem primären Amin der Formel (III) 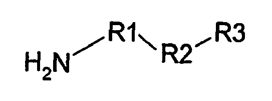 worin R1, R2 und R3 eine der oben angegebenen Bedeutung besitzen.

Diese Umsetzung kann man entweder ohne Lösungsmittel oder zweckmäßigerweise in einem inerten organischen Lösungsmittel, beispielsweise einem kurzkettigen Alkohol wie Methanol oder Ethanol, oder einem zyklischen gesättigten Ether, wie Tetrahydrofuran oder Dioxan, durchführen.

Gewöhnlich wählt man eine Reaktionstemperatur im Bereich von 20 bis 190 °C, insbesondere in einem Bereich von 60 bis 90 °C. Die Umsetzung ist in der Regel innerhalb von 1 bis 10 Stunden beendet.

Ein weiterer Gegenstand der vorliegenden Erfindung betrifft ein alternatives Verfahren zur Herstellung erfindungsgemäßer Verbindungen der Formel (I) zunächst durch Umsetzung einer Verbindung der Formel (II) 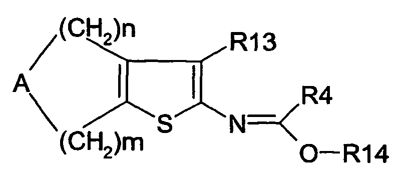 worin A, n, m und R4 eine der in Anspruch 1 angegebenen Bedeutung besitzen; R13 für CN oder C₁₋₃-Alkyl-O-CO- steht und R14 für C₁₋₃-Alkyl steht,
mit einem primären Amin der Formel (IV)

H₂N-R1-OH

worin R1 eine der oben angegebenen Bedeutung besitzt.

Diese Umsetzung führt man ähnlich wie obige Umsetzung in einem inerten Lösungsmittel, vorzugsweise Alkoholen wie Ethanol, bei einer Reaktionstemperatur im Bereich von 60 bis 120 °C durch.

Anschließend wird die terminale Hydroxylfunktion des zyklischen Produkts halogeniert. Dies kann durch Umsetzung der resultierenden Verbindung der Formel (V) 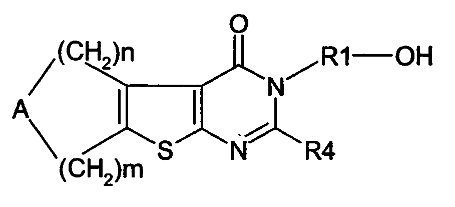 worin A, n, m, R4 und R1 eine der oben angegebenen Bedeutung besitzen,
mit einem Halogenierungsmittel, wie Thionylchlorid, erfolgen.

Weitere geeignete Halogenierungsmittel, wie Bromwasserstoffsäure sind dem Fachmann bekannt. Die Umsetzung wird entweder ohne Lösungsmittel oder zweckmäßigerweise in einem organischen Lösungsmittel, wie einem halogenierten Kohlenwasserstoff, bei einer Reaktionstemperatur von bis zu 100 °C durchgeführt.

Schließlich wird das halogenierte Produkt in die gewünschte Verbindung der Formel (I) überführt. Dies gelingt durch Umsetzung der resultierenden Verbindung der Formel (VI) 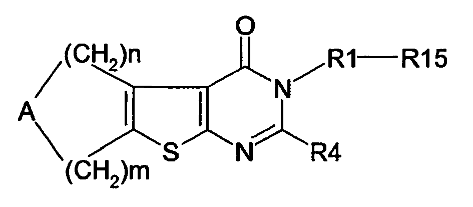 worin A, n, m, R4 und R1 eine der oben angegebenen Bedeutung besitzen und R15 für Halogen steht,
mit einem sekundären Amin der Formel (VII)

H-R2-R3

worin R2 und R3 eine der oben angegebenen Bedeutung besitzen. Diese Variante setzt voraus, dass der Rest R2 einen sekundären Stickstoff aufweist, über den der Rest R2 an den Rest R1 gebunden werden soll. Diese Umsetzung gelingt vorteilhafterweise in einem inerten organischen Lösungsmittel, vorzugsweise Toluol, Xylol, Dimethylformamid oder N-Methylpyrrolidon, in Gegenwart einer Base wie Kaliumcarbonat, Kaliumhydroxid oder Diisopropylethylamin bei einer Reaktionstemperatur im Bereich von RT bis 150 °C.

Die erfindungsgemäßen Verbindungen der Formel (I) können genauso wie die gegebenenfalls anfallenden Zwischenprodukte der Formeln (II), (V) und (VI) auf herkömmliche Art und Weise gewonnen sowie erforderlichenfalls aufgereinigt werden, beispielsweise durch Umkristallisieren aus üblichen organischen Lösungsmitteln, vorzugsweise einem kurzkettigen Alkohol wie Ethanol, oder mit Hilfe chromatographischer Techniken.

Je nach Einsatzstoffen fallen die erfindungsgemäßen Verbindungen der Formel (I) in freier Form oder bereits als Säureadditionssalze an. Sowohl die Verbindungen in freier Form als auch verfahrensgemäß resultierende Salze dieser Verbindungen können in an sich bekannter Weise in gewünschte Säureadditionssalze bzw. in die freie Form überführt werden.

Die erfindungsgemäßen Verbindungen besitzen nützliche pharmakologische Eigenschaften. Insbesondere weisen sie eine hohe Affinität zu 5-HT_{1B/D}- und 5-HT_{1A}-Rezeptoren auf.

Eine pharmakologisch besonders wertvolle Klasse erfindungsgemäßer Verbindungen ist dadurch gekennzeichnet, dass sie die natürliche durch Serotonin an 5-HT_{1B/D}- und 5-HT_{1A}-Rezeptoren hervorgerufenen Effekte antagonisieren.

Aufgrund ihrer pharmakologischen Eigenschaften sind die erfindungsgemäßen Verbindungen als Wirkstoffe für therapeutische Zwecke brauchbar.

Ein weiterer Gegenstand der vorliegenden Erfindung sind daher auch Mittel, insbesondere pharmazeutische Mittel, die wenigstens eine erfindungsgemäße Verbindung und erforderlichenfalls physiologisch akzeptable Hilfsstoffe enthalten.

Physiologisch akzeptabel sind die im Bereich der Pharmazie und angrenzenden Gebieten bekanntermaßen verwendbaren Hilfstoffe, insbesondere die in einschlägigen Arzneibüchern (z.B. DAB, Ph. Eur., BP, NF, USP) gelisteten, und auch andere Hilfstoffe, deren Eigenschaften einer physiologischen Anwendung nicht entgegenstehen.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Beispiele geeigneter pharmazeutischer Mittel sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Wirkstoffe verwendet werden. Ferner können auch Liposomen oder Mikrosphären zur Anwendung kommen.

Die Mittel können beispielsweise auf üblichem Wege verabreicht werden.

Bei der Herstellung der Mittel werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvörgangen, durchgeführt werden, z.B. eine Granulierung, Komprimierung und ähnliches.

Die erfindungsgemäße Verwendung zu therapeutischen Zwecken betrifft insbesondere die Behandlung von Störungen des Zentralen Nervensystems. Darunter versteht man Störungen, die vor allem das Gehirn betreffen. Der Begriff "Störung" im erfindungsgemäßen Sinne bezeichnet Anomalien, die in der Regel als krankhafte Zustände angesehen werden und sich in Form bestimmter Anzeichen, Symptome und/oder Fehlfunktionen zu erkennen geben können. Die erfindungsgemäße Behandlung kann auf einzelne Störungen sprich Anomalien bzw. krankhafte Zustände gerichtet sein, es können aber auch mehrere gegebenenfalls ursächlich miteinander verbundene Anomalien zu Mustern, d.h. Syndromen, zusammengefaßt sein, die erfindungsgemäß behandelt werden können.

Zu den erfindungsgemäß behandelbaren Störungen gehören vor allem neurologische und psychiatrische Störungen.

Zu neurologischen Störungen zählen neurodegenerative Störungen, insbesondere mit Alterungsprozessen, demyelinisierenden Prozessen, ischämischen Ereignissen und/oder weiteren morphologischen Veränderungen, in Zusammenhang stehende neurodegenerative Störungen. Zu den morphologischen Veränderungen gehören insbesondere diejenigen, die mit neuronalen Veränderungen und insbesondere Defiziten einhergehen, z.B. mit Infektionen, Traumata, Tumore, Ablagerungen und/oder diffusen himatrophischen Veränderungen, in Zusammenhang stehende neurodegenerative Störungen. Zu erfindungsgemäß behandelbaren neurologischen Störungen gehören Beeinträchtigungen mentaler Funktionen, vor allem Demenz, insbesondere cerebrovaskuläre Demenz und Demenz vom Alzheimer-Typ, z.B. senile Demenz und Alzheimer-Erkrankung, insbesondere intellektuelle Defizite, wie Aufmerksamkeitsstörungen (attention deficit disorders), amnesische und kognitive Störungen, z.B. Lern- und Gedächtnisschwäche (impaired cognitive function); Multiple Sklerose; Parkinson; Epilepsie; Delirium; Störungen von Aufmerksamkeit und Wach/Schlafverhalten, insbesondere Verhaltensstörungen und emotionale Störungen, deren Beginn in der Kindheit und Jugend liegt, wie Hyperaktivität bei Kindern; Narkolepsie und Schlafstörungen, z.B. restless legs syndrome; Entwicklungsstörungen.

Zu psychiatrischen Störungen zählen Psychosen, z.B. vom akuten exogenen Reaktionstyp oder Begleit-Psychosen organischer bzw. exogener Ursache, z.B. nach Trauma, vor allem Hirnläsionen und diffusen Himschädigungen, bei Stoffwechseistörungen, Infektionen und Endokrinopathien; endogene Psychosen, wie Schizophrenie sowie schizotype und wahnhafte Störungen; affektive Störungen, wie Depressionen, Manie bzw. manisch-depressive Zustände; sowie Mischformen der zuvor geschilderten Störungen; neurotische und somatoforme Störungen sowie Störungen bei Belastung; dissoziative Störungen, z.B. Bewußtseinsausfälle, -eintrübungen und -spaltungen und Persönlichkeitsstörungen; Angstzustände; Störungen des Sexuallebens, z.B. Impotenz des Mannes; depressive Zustände bei weiteren Erkrankungen, z.B. in Zusammenhang mit Fibromyalgie und Chronic Fatigue Syndrome; Eßstörungen, z.B. Anorexie oder Bulimie; und weitere nicht näher bezeichnete psychiatrische Störungen.

Eine besonders bevorzugte Ausführungsformen der vorliegenden Erfindung richtet sich auf die Behandlung von Depressionen.

Durch die erfindungsgemäße Behandlung lassen sich eine Vielzahl von Anzeichen, Symptomen und/oder Fehlfunktionen behandeln, die mit den Störungen und insbesondere den vorstehend genannten Zuständen zusammenhängen. Hierzu gehören beispielsweise dementielle Symptome, insbesondere solche mit Auswirkungen auf soziale Bezüge, eine Abnahme intellektueller Funktionen, z.B. Verwirrtheit, vor allem zeitlich und räumlich, Störungen der Merk- und Kombinationsfähigkeit sowie des Abstraktions- und Beurteilungsvermögens, ein gestörter Realitätsbezug, mangelnde Einsicht und Fähigkeit, üblichen sozialen Normen bzw. Lebensanforderungen zu genügen, Wesensveränderungen, Veränderungen der Einzeltriebe, wie Hunger, Schlaf, Durst, etc., und der Stimmungslage, Persönlichkeitsveränderungen, insbesondere Affektlabilität, Halluzinationen, Ich-Störungen, Zerfahrenheit, Ambivalenz, Autismus, Depersonalisation bzw. Sinnestäuschungen, Wahnideen, skandierende Sprache, fehlende Mitbewegung, kleinschrittiger Gang, Beugehaltung von Rumpf und Gliedern, Tremor, Mimikarmut, monotone Sprache, Depressionen, Apathie, erschwerte Spontaneität und Entschlußkraft, verarmte Assoziationsfähigkeit, Angst, nervöse Unruhe, Stottern, soziale Phobie, Panikstörungen, maniforme Syndrome, Erregungs- und Verwirrtheitszustände, Dysphorie, dyskinetische Syndrome und Tic-Störungen, z.B. bei Chorea-Huntington, Gilles-de-la-Tourette-Syndrom, Schwindelsyndrome, z.B. peripherer Lage-, Dreh- und Schwankschwindel, Melancholie, Hysterie, Hypochondrie und ähnliches.

Die erfindungsgemäße Verwendung der erfindungsgemäßen Wirkstoffe beinhaltet im Rahmen der Behandlung ein Verfahren. Dabei wird dem zu behandelnden Individuum, vorzugsweise einem Säuger, insbesondere einem Menschen, und auch einem Nutz- oder Haustier, eine wirksame Menge wenigstens einer Verbindung der Formel (I), in der Regel der pharmazeutischen Praxis entsprechend formuliert, verabreicht.

Die Erfindung betrifft auch die Herstellung von Mitteln zur Behandlung eines Individuums, vorzugsweise eines Säugers, insbesondere eines Menschen, Nutz- oder Haustieres.

Die folgenden Beispiele veranschaulichen die Erfindung, ohne sie einzuschränken. Es ist zu beachten, dass Bezeichnung und formelmäßige Darstellung von Salzen mit protoniertem Stickstoff lediglich eine von mehreren allesamt erfassten Möglichkeiten hinsichtlich der Ladungsverteilung wiedergeben. Dies gilt auch für tautomere Formen.
A Herstellung der Ausgangsmaterialien der Formel (II), (V) und (VI)
   Die als Ausgangsmaterialien der Formel (II) eingesetzten 2-Amino-3-carboethoxy-(cyano)-4,5,6,7-tetrahydro-thieno[2,3-c]pyridine mit 6-ständiger Methyl-, Benzyl-, Acetyl-, Benzoyl-Gruppe oder mit unsubstituierter 6-Position sind literaturbekannt (K. Gewald et al, Chem. Ber. 99, 94-100 (1966)). Die Herstellung dieser Ausgangsmaterialien ist auch in WO 98/11110; WO 98/56793 beschrieben.
A1) 2-Ethoxymethylen-amino-3-carboethoxy-6-methyl-4,5,6,7-tetrahydro-thieno[2,3-c]pyridin
   (Ausgangsmaterial der Formel (II))
   40,0 g (167 mM) 2-Amino-3-carboethoxy-6-methyl-4,5,6,7-tetrahydro-thieno[2,3-c]pyridin in 250 ml Triethylorthoformiat wurden mit 3,2 ml Acetanhydrid versetzt und unter Stickstoff 3 h am Rückfluß gekocht. Danach engte man den Ansatz bei 80°C am Rotationsverdampfer ganz ein. Man isolierte 48,0 g (97 %) Rohprodukt als dunkles Öl, das für die weitere Umsetzung genügend rein ist.
A2) 3-(2-Hydroxyethyl)-7-methyl-5,6,7,8-tetrahydropyrido[4',3':4,5]thieno[2,3-*d*]pyrimidin-4(3H)-on
   (Ausgangsmaterialien der Formel (V))
   86,4 g (292 mM) 2-Ethoxymethylen-amino-3-carboethoxy-6-methyl-4,5,6,7-tetrahydro-thieno[2,3-c]pyridin in 200 ml Ethanol wurden mit 17,6 ml (292 mM) Ethanolamin versetzt und 2 h am Rückfluß gekocht. Anschließend engte man den Ansatz im Vakuum ein und nahm den Rückstand unter Rühren in 30 ml Essigester auf. Die über Nacht ausgefallenen Festkörper saugte man ab und wusch mit wenig Essigester nach. Nach Umkristallisieren aus Ethanol isolierte man 48,0 g (62 %) Produkt mit Schmp. 163-165°C.
A3) 3-(2-Chloroethyl)-7-methyl-5,6,7,8-tetrahydropyrido[4',3':4,5]thieno[2,3-*d*]pyrimidin-4(3H)-on
   (Ausgangsmaterial der Formel (VI))
   42,0 g (158 mM) 3-(2-Hydroxyethyl)-7-methyl-5,6,7,8-tetrahydropyrido[4',3':4,5]thieno[2,3-*d*]pyrimidin-4-on in 240 ml 1,2-Dichlorethan wurden auf Rückfluß erhitzt und anschließend 12,7 ml (175 mM) Thionylchlorid in 20 ml 1,2-Dichlorethan zugetropft. Nach 2 h Rückflußkochen ließ man das Reaktionsgemisch abkühlen und goß auf Eis/Wasser. Man verteilte bei pH = 10 zwischen Methylenchlorid und Wasser und extrahierte die wäßrige Phase mit Methylenchlorid nach. Nach Trocknen engte man die vereinigten organischen Phasen ein. Das Rohprodukt (40 g) kristallisierte man aus 400 ml Isopropanol um. Man isolierte 30,5 g (68 %) Produkt mit Schmp. 159-161°C.
Analog A1) bis A3) stellte man her:
A4) 3,4,5,6,7,8-Hexahydro-3-(1-hydroxy)-prop-2-yl-7-methyl-pyrido[4',3':4,5]thieno[2,3-d]pyrimidin-4-on
   (Ausgangsmaterial der Formel (V))
A5) 3,4,5,6,7,8-Hexahydro-3-(1-chlor)-prop-2-yl-7-methyl-pyrido[4',3':4,5]thieno[2,3-d]pyrimidin-4-on
   (Ausgangsmaterial der Formel (VI))
A6) 3-(2-Hydroxyethyl)-3,5,6,8-tetrahydro-4H-pyrano[4',3':4,5]thieno[2,3-*d*]pyrimidin-4(3H)-on (ausgehend von Tetrahydro-4H-pyran-4-on)
   (Ausgangsmaterial der Formel (V))
A7) 3-(2-Chlorethyl)-3,5,6,8-tetrahydro-4H-pyrano[4',3':4,5]thieno[2,3-*d*]pyrimidin-4(3H)-on
   (Ausgangsmaterial der Formel (VI))
A8) 3-(2-Hydroxyethyl)-3,5,6,8-tetrahydro-4H-thiopyrano[4',3':4,5]thieno[2,3-*d*]pyrimidin-4(3H)-on (ausgehend von Tetrahydro-4H-thiopyran-4-on)
   (Ausgangsmaterial der Formel (V))
A9) 3-(2-Chlorethyl)-3,5,6,8-tetrahydro-4H-thiopyrano[4,3':4,5]thieno[2,3-*d*]pyrimidin-4(3H)-on
   (Ausgangsmaterial der Formel (VI))
B) Herstellung der Ausgangsmaterialien der Formel (VII):
B1) 3-Piperazin-1-yl-5-methyl-1,2-benzisoxazol *3-Chlor-5-methyl-1,2-benzisoxazol* 3-Hydroxy-5-methyl-1,2-benzisoxazol (15,0 g, 100,6 mmol; Herstellung: Australian Journal of Chemistry (1977), 30(8), 1847-50) wurden in Triethylamin (15,4 ml, 110,6 mmol) suspendiert, und zu dieser Mischung wurde unter Kühlung Phosphoroxytrichlorid (37,0 g, 22,5 ml, 241,4 mmol) bei Raumtemperatur zugetropft. Da nach etwa der Hälfte der zugetropften Phosphoroxytrichlorid-Menge die Reaktionsmischung kaum mehr rührbar war, wurde die Temperatur auf 50°C erhöht. Nach Zutropfen der gesamten Menge Phosphoroxytrichlorid erhitzte man die Reaktionsmischung noch 5h auf 130°C, wonach laut DC die Umsetzung beendet war. Nach Abkühlen wurde der Ansatz langsam unter Kühlung in Wasser (200 ml) getropft, die wässrige Phase wurde 3-mal mit MtB-Ether extrahiert, die organischen Phasen wuden mit Wasser gewaschen, über Magnesiumsulfat getrocknet und im Vakuum einrotiert. Dabei wurden 14,6 g 3-Chlor-5-methyl-1,2-benzisoxazol (Ausbeute: 87%) als zähflüssiges Öl erhalten, welches über Nacht zu einem Festprodukt erstarrte.
   MS (MSD) *m*/*z* 168 [M+H⁺].
   *3-Piperazin-1-yl-5-methyl-1,2-benzisoxazol*
   3-Chlor-5-methyl-1,2-benzisoxazol (2,17 g, 12,93 mmol) wurde zusammen mit Piperazin (10,0 g, 116,1 mmol) und DBU (Diazbicycloundecen; 2,0 g 13,14 mmol) in einer Schmelze 4h bei 120°C gerührt, wonach laut DC die Umsetzung beendet war. Nach Abkühlen wurde der Ansatz in Wasser gegeben, die wässrige Phase wurde 3-mal mit Dichlormethan extrahiert, die organischen Phasen wurden mit Wasser gewaschen, über Magnesiumsulfat getrocknet und im Vakuum einrotiert. Dabei wurden 2,24 g 3-Piperazin-1-yl-5-methyl-1,2-benzisoxazol (Ausbeute: 80%) als leicht verunreinigtes Festprodukt erhalten, welches durch Chromatographie aufgereinigt wurde.
   MS (MSD) *m*/*z* 218 [M+H⁺].
B2) 3-Piperazin-1-yl-7-methyl-1,2-benzisoxazol
   Analog Boeshagen et al., Chem. Ber. 100, 10, 1967, 3326-3330 wurde 7-Methyl-1,2-benzisoxazol (5g, 33.52 mmol) in die entsprechende 3-Chloro-Verbindung umgesetzt (0.7 g Öl), und anschließend analog zur Darstellung von 3-Piperazin-1-yl-1,2-benzisoxazol in das entsprechende 3-Piperazin-1-yl-7-methyl-1,2-benzisoxazol überführt (0.18 g gelbliches Öl, MS (ESI) m/z 218 [M+H⁺]).
B3) 3-Piperazin-1-yl-5-methoxy-1,2-benzisoxazol:
   Nach Boeshagen et al., Chem. Ber. 100, 10, 1967, 3326-3330 hergestelltes 5-Methoxy-3-chloro-1,2-benzisoxazol (4.7 g, 25.6 mmol), Piperazin (19.8 g, 229.86 mmol) und 6ml DBU wurden 2h lang auf 120°C erhitzt, anschließend in CH₂Cl₂ aufgenommen, mit H₂O gewaschen, getrocknet und eingedampft. Der so erhaltene Rückstand (6.1 g braunes Öl) wurde erst mit MTB und anschließend mit MTB/n-Pentan 1:1 verrührt, und ergab 5.9 g amorphen Festkörper (MS (ESI) m/z 234.25 [M+H⁺]).
B4) 5-Methoxy-1-methyl-3-piperidin-4-yl-1H-indol
   Die Darstellung erfolgte gemäß WO 02/50067 (S.56).
B5) 4-(5-Methyl-1-benzofuran-3-yl)piperidin
   Die Darstellung erfolgte durch Umsetzung von 4-Chloranisol mit 1-Acetylpiperidine-4-carbonylchlorid zum Zwischenprodukt (1-Acetylpiperidin-4-yl)-(5-chloro-2-hydroxyphenyl)methanon analog Strupczewski et al., J. Med. Chem. 1985, 28, 761-69. Nach Alkylierung der OH-Funktion mit Bromessigsäuremethylester wurde analog Kaltenbronn et al, Eur. J. Med. Chem. 1997, 32 (5), 425-431 zum Methyl-3-(1-acetylpiperidin-4-yl)-5-methyl-1-benzofuran-2-carboxylat zyklisiert. Nach der Verseifung der Esterfunktion, Decarboxylierung und anschließender Hydrolyse der Acetylgruppe wurde 4-(5-methyl-1-benzofuran-3-yl)piperidin erhalten (Zur Herstellung siehe auch DE 2537837).
B6) 4-(5-Chloro-1-benzofuran-3-yl)piperidin
   Die Darstellung erfolgte analog der Darstellung des 4-(5-Methyl-1-benzofuran-3-yl)piperidins (s. B5).
B7) 3-Piperidin-4-yl-1H-indol
   Ist kommerziell erhältlich.
B8) 3-Piperidin-4-yl-1H-pyrrolo[2,3-b]pyridin
   Die Herstellung erfolgte durch Hydrierung von 3-(1,2,3,6-Tetrahydropyridin-4-yl)-1H-pyrrolo[2,3-b]pyridin in Ethanol mit Palladium auf Kohle unter Zusatz von Salzsäure.
B9) 3-(1,2,3,6-Tetrahydropyridin-4-yl)-1H-indol
   Die Herstellung erfolgte gemäß DE 2738646 (S.15).
B10) 3-(1,2,3,6-Tetrahydropyridin-4-yl)-1H-pyrrolo[2,3-b]pyridin
   Die Herstellung erfolgte gemäß WO 00/64898 (S.7).
B11) 4-[5-(4-Chlorophenyl)-1 H-pyrazol-3-yl]piperidin
   Ist kommerziell erhältlich
B12) 4-[5-(2-Thienyl)-1H-pyrazol-3-yl]piperidin
   Ist kommerziell erhältlich.
B13) 1-Piperidin-4-yl-1H-1,2,3-benzotriazol
   Ist kommerziell erhältlich.
B14) 1-Piperidin-4-yl-1H-benzimidazol
   Ist kommerziell erhältlich
B15) 1-(1-Phenyl-1H-tetrazol-5-yl)piperazin
   Die Herstellung erfolgte durch Umsetzung von 5-Chlor-1-phenyl-1H-tetrazol mit tert-Butyl-piperazin-1-carboxylat in DMF bei 40°C.
C Herstellung der Endprodukte der Formel (I)

Sofern nicht anderes angegeben ist, erfolgte die Herstellung der folgenden Verbindungen durch Umsetzung geeigneter Ausgangsmaterialien der Formel (VI) mit geeigneten Ausgangsmaterialien der Formel (VII), wie in Beispiel 9 detailliert erläutert ist:

### Beispiel 1:

4-(5-methyl-1,2-benzisoxazol-3-yl)-1-[2-(4-oxo-5,8-dihydro-4H-pyrano[4',3':4,5]thieno[2,3-d]pyrimidin-3(6H)-yl)ethyl]piperazin-1-iumfumarat 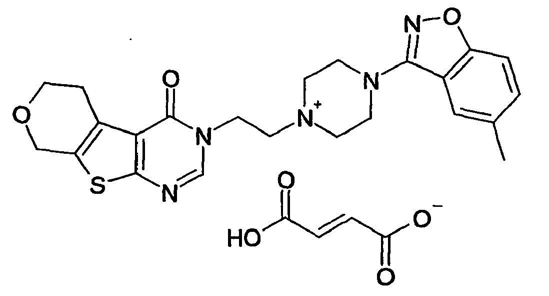 ESI-MS [M+H⁺] = 452.
¹³C-NMR (100.6 MHz, DMSO-*d₆*) δ: 20.5, 26.0, 42.3, 47.7 (2 C), 51.9 (2 C), 55.8, 63.8, 64.2, 109.5, 115.6, 121.3, 122.0, 128.4, 130.4, 131.2, 131.9, 148.2, 156.9, 160.6, 161.7, 162.2 ppm. (plus zwei Fumaratsignale).

### Beispiel 2:

3-{2-[4-(5-methyl-1,2-benzisoxazol-3-yl)piperazin-1-yl]ethyl}-3,5,6,8-tetrahydro-4H-thiopyrano[4',3':4,5]thieno[2,3-d]pyrimidin-4-on 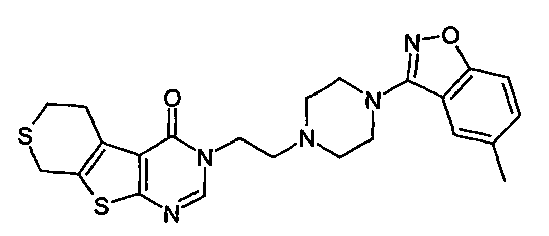 ESI-MS [M+H⁺] = 468.
¹³C-NMR (100.6 MHz, DMSO-*d₆*) δ: 20.3, 24.5, 24.6, 27.2, 45.0 (2 C), 50.7 (3 C), 54.3, 109.4, 115.1, 121.6, 121.8, 129.2, 130.4, 131.3, 131.9, 147.7, 157.0, 159.5, 160.8, 161.8 ppm.

### Beispiel 3:

3-{(1S)-1-methyl-2-[4-(5-methyl-1,2-benzisoxazol-3-yl)piperazin-1-yl]ethyl}-3,5,6,8-tetrahydro-4H-pyrano[4',3':4,5]thieno[2,3-d]pyrimidin-4-on 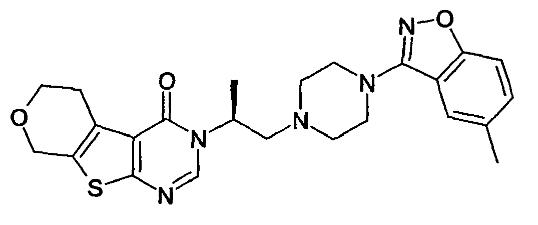 ESI-MS [M+H⁺] = 466.
¹³C-NMR (100.6 MHz, DMSO-*d₆*) δ: 18.1, 20.4, 26.0 (2C), 47.6 (2C), 52.0 (2C), 60.9, 63.8, 64.2, 109.5, 115.5, 120.9, 122.0, 128.6, 130.4, 131.1, 131.8, 145.8, 156.9, 160.4, 161.6, 161.7 ppm.

### Beispiel 4:

3-{2-[4-(1,2-Benzisothiazol-3-yl)piperazin-1-yl]ethyl}-7-methyl-5,6,7,8-tetrahydropyrido[4',3':4,5]thieno[2,3-*d*]pyrimidin-4(3*H*)-on 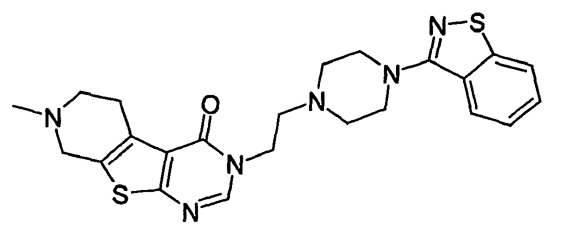 Fp: 82-84°C; ESI-MS [M+H⁺] = 467.

### Beispiel 5:

3-{2-[4-(MChloro-1, 2-benzisothiazol-3-yl)piperazin-1-yl]ethyl}-7-methyl-5,6,7,8-tetrahydropyrido[4',3':4,5]thieno[2,3-*d*]pyrimidin-4(3*H*)-on 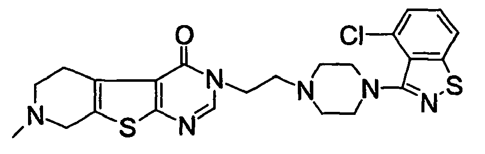 Fp: 88-89°C; ESI-MS [M+H⁺] = 501.

### Beispiel 6:

7-Methyl-3-(2-{4-[5-(trifluoromethyl)-1,3-benzothiazol-2-yl]piperazin-1-yl}ethyl)-5,6,7,8-tetrahydropyrido[4',3':4,5]thieno[2,3-*d*]pyrimidin-4(3*H*)-on 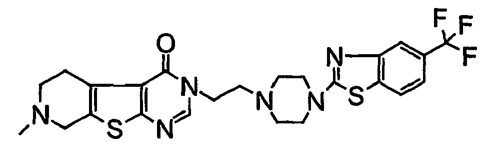 Fp: 166-67°C; ESI-MS [M+H⁺] = 535.

### Beispiel 7:

3-{2-[4-(6-Chloro-1,3-benzothiazol-2-yl)piperazin-1-yl]ethyl}-7-methyl-5,6,7,8-tetrahydropyrido[4',3':4,5]thieno[2,3-*d*]pyrimidin-4(3H)-on 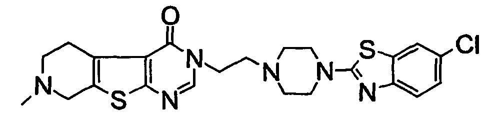 Fp: 160-162°C; ESI-MS [M+H⁺] = 501.

### Beispiel 8:

3-{2-[4-(1*H*-Benzimidazol-2-yl)piperazin-1-yl]ethyl}-7-methyl-5,6,7,8-tetrahydropyrido[4',3':4,5]thieno[2,3-*d*]pyrimidin-4(3*H*)-on 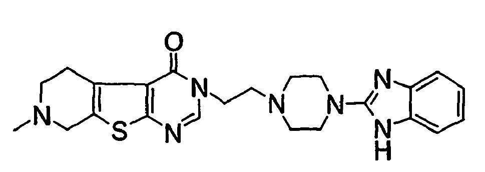 Fp: 227-229°C; ESI-MS [M+H⁺] = 450.

### Beispiel 9:

7-Methyl-3-{2-[4-(5-methyl-1,2-benzisoxazol-3-yl)piperazin-1-ium-1-yl]ethyl}-4-oxo-3,4,5,6,7,8-hexahydropyrido[4',3':4,5]thieno[2,3-*d*]pyrimidin-7-iumdichlorid 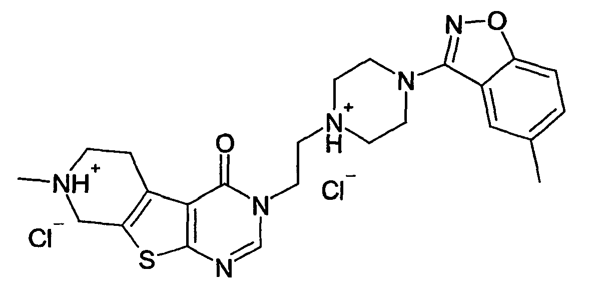 3-(2-Chloroethyl)-7-methyl-5,6,7,8-tetrahydropyrido[4',3':4,5]thieno[2,3-d]pyrimidin-4(3H)-one (2,5 g, 8,81 mol; Herstellung: Beispiel A3) wurde zusammen mit 3-Piperazin-1-yl-5-methyl-1,2-benzisoxazol (2,49 g, 11,45 mmol), Diisopropylethylamin (15,4 ml, 88,1 mmol), Natriumbromid (4,53 g, 44,05 mmol) und N-Methyl-2-pyrrolidon (40ml) 4 Tage bei 70°C gerührt. Nach Abkühlen wurden im Hochvakuum N-Methyl-2-pyrrolidon und überschüssiges Diisopropylethylamin entfernt, der Rückstand wurde in Wasser gegeben, die wässrige Phase wurde mehrfach mit Essigester extrahiert, und die organischen Phasen wurden mit Wasser gewaschen, über Magnesiumsulfat getrocknet und im Vakuum einrotiert. Das stark verunreinigte Rohprodukt wurde über Kieselgel chromatographiert (Elutionsmittel: Methylenchlorid mit langsam steigendem Methanolanteil (0-50%)). Dabei wurden 0,9 g 7-Methyl-3-{2-[4-(5-methyl-1,2-benzisoxazol-3-yl)piperazin-1-yl]ethyl}-5,6,7,8-tetrahydropyrido[4',3':4,5]thieno[2,3-d]pyrimidin-4(3H)-on (Ausbeute: 22%) als Festprodukt erhalten, welches mittels etherischer Salzsäure in das entsprechende Dihydrochlorid überführt wurde.
¹³C-NMR (125.69 MHz, DMSO-*d₆*) δ: 163.1, 161.8, 159.7, 157.3, 148.6, 132.3, 131.7, 127.8, 124.3, 121.7, 120.6, 115.1, 109.7, 54.0, 50.6(2C), 50.4, 49.6, 44.7 (2C), 41.8, 40.1, 22.7, 20.5 ppm.
ESI-MS [M+H+] = 465.

### Beispiel 10:

3-{2-[4-(1,3-8enzoxazol-2-yl)piperazin-1-yl]ethyl}-7-methyl-5,6,7,8-tetrahydropyrido[4',3':4,5]thieno[2,3-d]pyrimidin-4(3*H*)-on 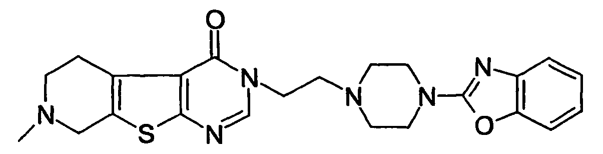 Fp: 130-132°C; ESI-MS [M+H⁺] = 451.

### Beispiel 11:

3-{2-[4-(1,3-Benzothiazol-2-yl)piperazin-1-yl]ethyl}-7-methyl-5,6,7,8-tetrahydropyrido[4',3':4,5]thieno[2,3-*d*]pyrimidin-4(3*H*)-on 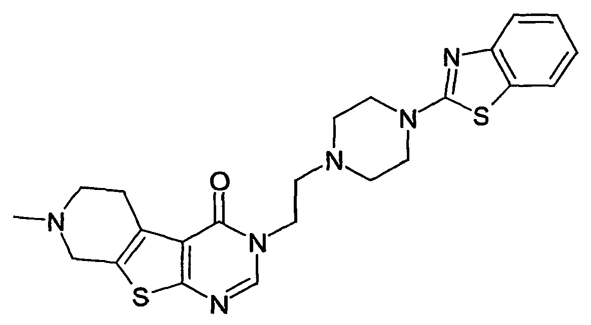 Fp: 151-153°C; ESI-MS [M+H⁺] = 467.

### Beispiel 12:

3-{2-[4-(1,2-Benzisoxazol-3-yl)piperazin-1-ium-1-yl]ethyl}-7-methyl-4-oxo-3,4,5,6,7,8-hexahydropyrido[4',3':4,5]thieno[2,3-*d*]pyrimidin-7-iumdichlorid 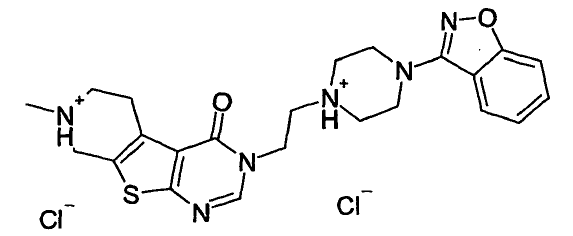 ESI-MS [M+H⁺] = 451.
¹H-NMR (500 MHz, DMSO-*d₆*) δ: 8.52 (m, 1 H), 8.04 (m, 1H), 7.63 (m ,2H), 7.34 (m, 1 H), 4.62 (m, 1 H), 4.45 (m, 3H), 4.20 (m, 2H), 3.80-3.20 (m, 12H, durch Wasser überlagert) 2.95 (s, 3H).

### Beispiel 13:

3-{2-[4-(1*H*-Indazol-3-yl)piperazin-1-yl]ethyl}-7-methyl-5,6,7,8-tetrahydropyrido[4',3':4,5]thieno[2,3-*d*]pyrimidin-4(3*H*)-on 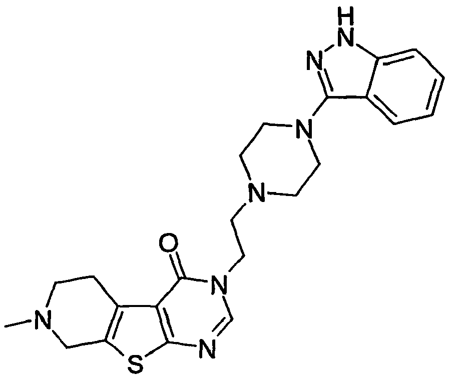 ESI-MS [M+H⁺] = 450.
¹H-NMR (500 MHz, DMSO-*d₆*) δ: 11.96 (s, 1 H), 8.31 (s, 1 H), 7.72 (d, 1 H), 7.33 (d, 1H), 7.27 (m, 1 H), 6.96 (m, 1 H), 4.11 (m, 2H), 3.56 (s, 2H), 3.35-3.25 (m, 4H, durch Wasser überlagert), 2.96 (m, 2H), 2.68-2.60 (m, 8H), 2.37 (s, 3H).

### Beispiel 14:

4-(7-Methyl-1,2-benzisoxazol-3-yl)-1-[2-(7-methyl-4-oxo-5,6,7,8-tetrahydropyrido[4,3':4,5]thieno[2,3-d]pyrimidin-3(4*H*)-yl)ethyl]piperazin-1-iumchlorid 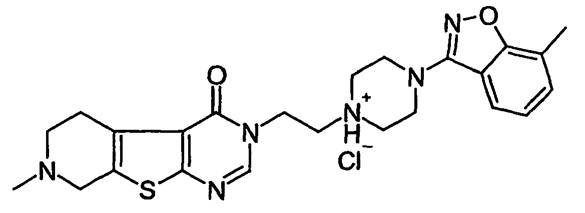 Die Titelverbindung wurde durch Umsetzung von 3-Piperazin-1-yl-7-methyl-1,2-benzisoxazol mit 3-(2-Chloroethyl)-7-methyl-5,6,7,8-tetrahydropyrido[4',3':4,5]thieno[2,3-*d*]pyrimidin-4-on und nachfolgender Reinigung durch Chromatographie an Kieselgel erhalten und anschließend in das entsprechende Fumarat überführt.(CH2C12/CH3OH 0-8%) erhalten; 70mg; MS (ESI) m/z 465 [M+H⁺].
¹H-NMR (500 MHz, DMSO-*d₆*) δ: 8.35 (s, 1 H), 7.78 (d, 1 H), 7.47 (d, 1H), 7.19 (m, 1 H), 6.61 (s, 2H), 4.12 (m, 2H), 3.65 (s, 2H), 3.44 (m, 4H), 2.98 (m, 2H), 2.77 (m, 2H), 2.68 (m, 6H), 2.45 (s, 3H), 2.42 (s, 3H).

### Beispiel 15:

4-(5-Methoxy-1,2-benzisoxazol-3-yl)-1-[2-(7-methyl-4-oxo-5,6,7,8-tetrahydropyrido[4',3':4,5]thieno[2,3-*d*]pyrimidin-3(4*H*)-yl)ethyl]piperazin-1-iumchlorid 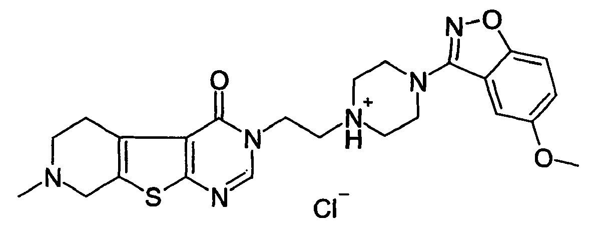 ¹³C-NMR (125.76 MHz, D₂O) δ: 163.8, 160.4, 159.2, 159.0, 155.3, 148.3, 127.8, 125.1, 121.4, 120.8, 114.9, 111.3, 103.2, 56.5, 55.4, 52.0 (2C), 51.9, 51.2, 45.5 (2C), 42.6, 41.5, 22.9 ppm.
ESI-MS [M+H⁺] = 481.

### Beispiel 16:

4-(5-Methoxy-1-methyl-1H-indol-3-yl)-1-[2-(7-methyl-4-oxo-5,6,7,8-tetrahydropyrido[4',3':4,5]thieno[2,3-d]pyrimidin-3(4H)-yl)ethyl]piperidiniumchlorid 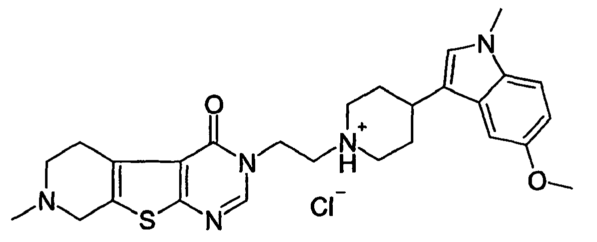 ESI-MS [M+H⁺] = 492.

### Beispiel 17

4-(5-Methyl-1-benzofuran-3-yl)-1-[2-(7-methyl-4-oxo-5,6,7,8-tetrahydropyrido[4',3':4,5]thieno[2,3-d]pyrimidin-3(4H)-yl)ethyl]piperidiniumchlorid 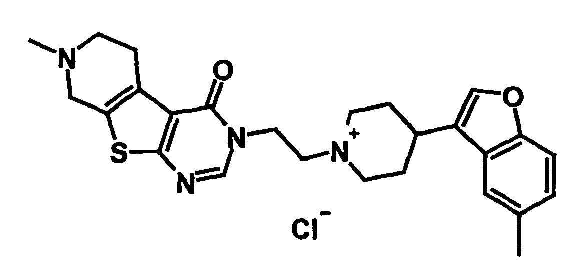 ¹³C-NMR (125.76 MHz, D₂O) δ: 163.6, 159.0, 153.3, 148.0, 141.0, 132.6, 127.5, 126.4, 125.8, 124.7, 122.4, 121.1, 119.4, 111.1, 55.0, 53.5 (2C), 51.5, 50.8, 42.1, 41.4, 29.3, 28.8 (2C), 22.5, 20.3 ppm.
ESI-MS [M+H⁺] = 483.

### Beispiel 18

4-(5-Chloro-1-benzofuran-3-yl)-1-[2-(7-methyl-4-oxo-5,6,7,8-tetrahydropyrido[4',3':4,5]thieno[2,3-d]pyrimidin-3(4H)-yl)ethyl]piperidiniumchlorid 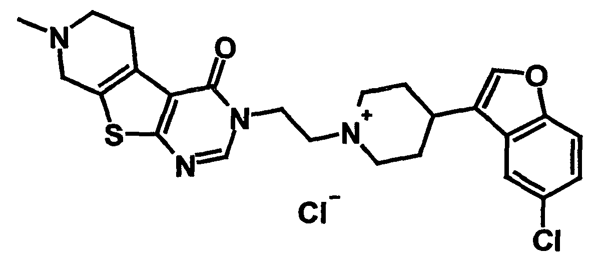 ¹³C-NMR (125.76 MHz, D₂O) δ: 163.6, 159.0, 1531.4, 148.0, 142.3, 127.7, 127.5, 127.4, 124.7, 124.5, 122.4, 121.0, 119.2, 112.6, 55.0, 53.4 (2C), 51.5, 50.8, 42.2, 41.4, 29.2, 28.8 (2C), 22.5 ppm.
ESI-MS [M+H⁺] = 483.

### Beispiel 19:

4-(1 H-Indol-3-yl)-1-[2-(7-methyl-4-oxo-5,6,7,8-tetrahydropyrido[4',3':4,5]thieno[2,3-d]pyrimidin-3(4H)-yl)ethyl]piperidiniumchlorid 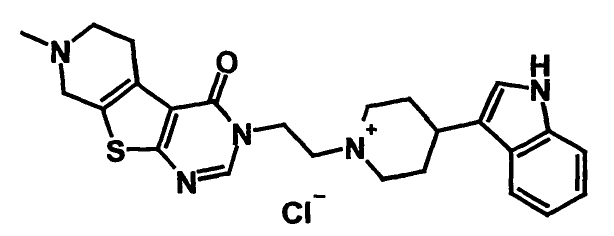 ¹³C-NMR (100,61 MHz, D₂O) δ :163.6, 158.9, 147.9, 136.2, 127.4, 125.4, 125.0, 124.7, 122.0, 121.1, 119.1, 118.5, 117.4, 111.8, 54.9, 53.7 (2C), 51.5, 50.8, 42.2, 41.4, 30.4, 29.8 (2C), 22.5 ppm.
ESI-MS [M+H⁺] = 448.

### Beispiel 20:

3-{2-[4-(1H-Indol-3-yl)-3,6-dihydropyridin-1(2H)-yl]ethyl)-7-methyl-5,6,7,8-tetrahydropyrido[4',3':4,5]thieno[2,3-d]pyrimidin-4(3H)-one 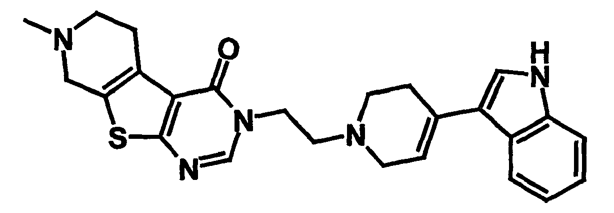 ¹³C-NMR (100,61 MHz, DMSO-*d₆*) δ: 161.9, 156.9, 148.0, 136.9, 130.3, 129.7, 128.9, 124.6, 122.7, 121.3, 121.1, 120.0, 119.1, 117.4, 115.8, 111.7, 55.7, 52.9 (2C), 51.2, 48.5, 44.9, 42.6, 28.4, 25.7 ppm.
ESI-MS [M+H⁺] = 446.

### Beispiel 21:

1-[2-(7-Methyl-4-oxo-5,6,7,8-tetrahydropyrido[4',3':4,5]thieno[2,3-d]pyrimidin-3(4H)-yl)ethyl]-4-(1H-pyrrolo[2,3-b]pyridin-3-yl)-1,2,3,6-tetrahydropyridiniumacetat 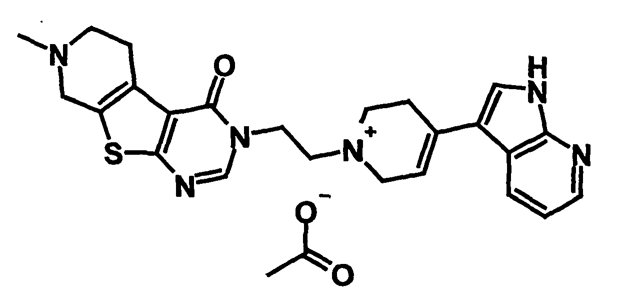 ¹³C-NMR (125,7 MHz, DMSO-*d₆*) δ: 162.9, 157.4, 149.0, 148.6, 142.9, 129.3, 128.1, 127.8, 124.5, 124.3, 120.7, 116.5, 115.9, 112.8 (2C), 53.7, 50.6, 50.5, 49.7, 48.6, 41.9, 40.5, 24.5, 22.9 ppm
ESI-MS [M+H⁺] = 447.

### Beispiel 22:

1-[2-(7-Methyl-4-oxo-5,6,7,8-tetrahydropyrido[4',3':4,5]thieno[2,3-d]pyrimidin-3(4H)-yl)ethyl]-4-(1H-pyrrolo[2,3-b]pyridin-3-yl)piperidiniumacetat 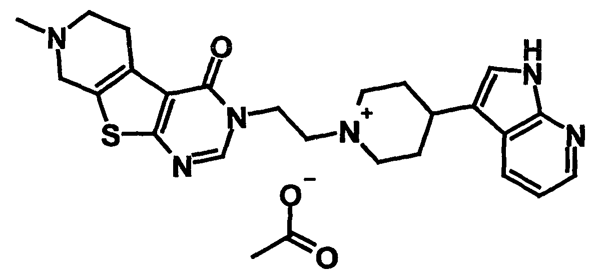 ¹³C-NMR (125,7 MHz, D₂O) δ: 164.0, 159.5, 148.4, 147.2, 142.1, 129.4, 127.9, 125.2, 122.2, 121.6, 119.6, 117.0, 115.7, 55.1, 53.7 (2C), 51.9, 51.2, 42.6, 41.9, 30.6, 29.7, 29.6, 23.0 ppm.
ESI-MS [M+H⁺] = 447.

### Beispiel 23:

4-[5-(4-Chlorophenyl)-1H-pyrazol-3-yl]-1-[2-(7-methyl-4-oxo-5,6,7,8-tetrahydropyrido[4',3':4,5]thieno[2,3-d]pyrimidin-3(4H)-yl)ethyl]piperidiniumchlorid 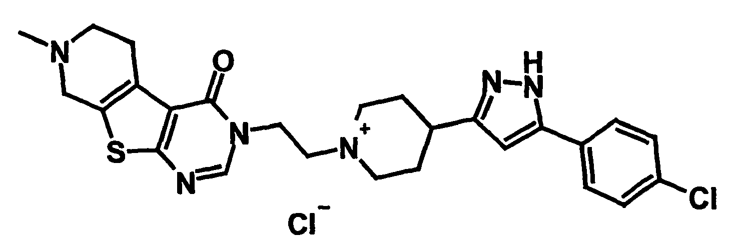 ¹³C-NMR (125,7 MHz, DMSO-*d₆*) δ: 161.2, 155.3, 148.2, 146.7, 144.1, 130.3, 128.7, 126.9 (2C), 126.1, 125.0 (2C), 122.6, 118.9, 97.6, 52.2, 49.9 (2C), 48.3. 47.6, 39.8, 38.5, 29.2, 26.4 (2C), 20.8 ppm
ESI-MS [M+H⁺] = 509.

### Beispiel 24:

1-[2-(7-Methyl-4-oxo-5,6,7,8-tetrahydropyrido[4',3':4,5]thieno[2,3-d]pyrimidin-3(4H)-yl)ethyl]-4-[5-(2-thienyl)-1H-pyrazol-3-yl]piperidiniumchlorid 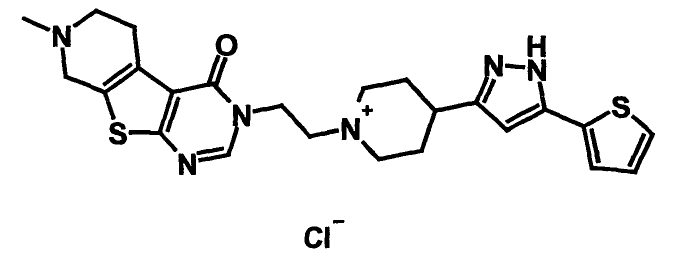 ¹³C-NMR (125,7 MHz, D₂O) δ: 166.7, 162.2, 153.1, 151.1, 146.5, 134.0, 131.3 (2C), 130.6, 129.6, 127.8, 124.3, 103.9, 58.1, 55.9, 54.5 (2C), 53.9, 45.2, 44.4, 33.7, 31.5 (2C), 25.6 ppm.
ESI-MS [M+H⁺] = 481.

### Beispiel 25:

3-{2-[4-(1H-1,2,3-Benzotriazol-1-yl)pipendinium-1-yl]ethyl}-7-methyl-4-oxo-3,4,5,6,7,8-hexahydropyrido[4',3':4,5]thieno[2,3-d]pyrimidin-7-iumdichlorid 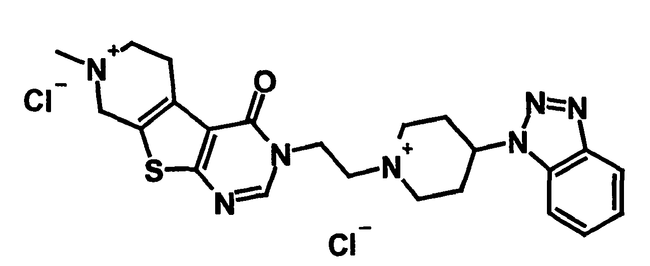 ¹³C-NMR (125,7 MHz, D₂O) δ: 163.9, 159.4, 148.4, 144.9, 132.7, 128.6, 127.9, 125.7, 122.2, 125.2, 121.5, 119.0, 110.6, 55.2, 53.1, 52.1, 51.9(2C), 51.2, 42.6, 42.0, 28.9, 22.9 ppm.
ESI-MS [M+H⁺] = 450.

### Beispiel 26:

4-(1H-Benzimidazol-1-yl)-1-[2-(7-methyl-4-oxo-5,6,7,8-tetrahydropyrido[4',3':4,5]thieno[2,3-d]pyrimidin-3(4H)-yl)ethyl]piperidiniumchlorid 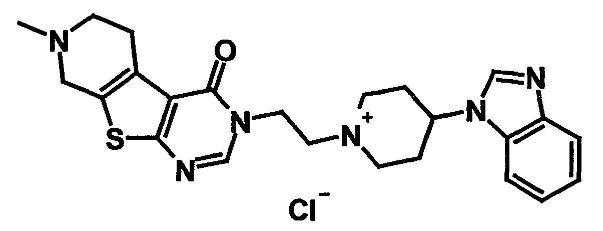 ESI-MS [M+H⁺] = 449.

### Beispiel 27:

1-[2-(7-Methyl-4-oxo-5,6,7,8-tetrahydropyrido[4',3':4,5]thieno[2,3-d]pyrimidin-3(4H)-yl)ethyl]-4-(1-phenyl-1H-tetrazol-5-yl)piperazin-1-iumchlorid 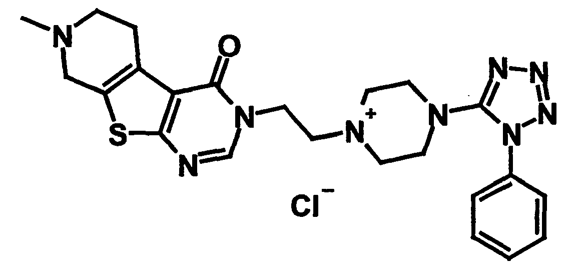 ESI-MS [M+H⁺] = 478.

### Beispiel 28:

3-{2-[4-(1-Benzothien-3-yl)-3,6-dihydropyridin-1(2H)-yl]ethyl}-7-methyl-5,6,7,8-tetrahydropyrido[4',3':4,5]thieno[2,3-d]pyrimidin-4(3H)-one

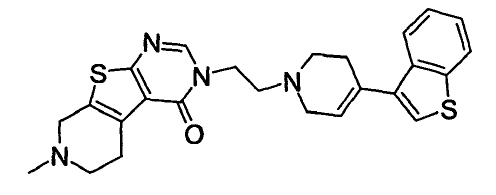

### Beispiel 29:

7-Methyl-3-{2-[4-(3-phenyl-1,2,4-thiadiazol-5-yl)piperazin-1-yl]ethyl}-5,6,7,8-tetrahydropyrido[4',3':4,5]thieno[2,3-d]pyrimidin-4(3H)-one

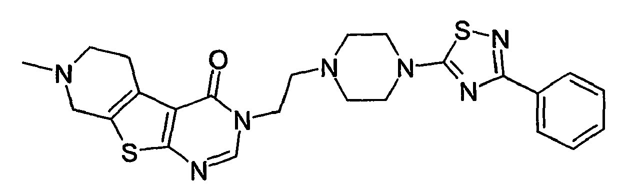

### Beispiel 30:

7-Methyl-3-{2-[4-(4-phenyl-1,3-thiazol-2-yl)piperazin-1-yl]ethyl}-5,6,7,8-tetrahydropyrido[4',3':4,5]thieno[2,3-d]pyrimidin-4(3H)-one

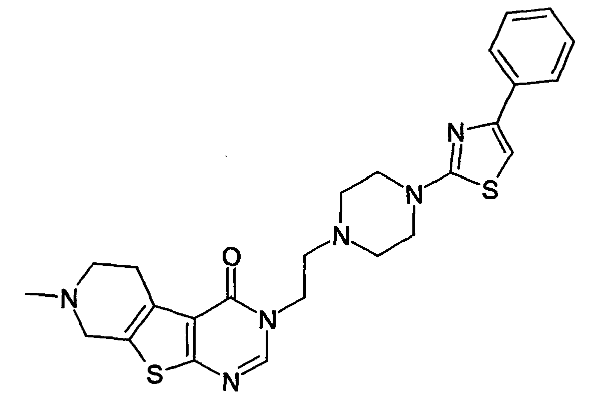

### Beispiel 31:

4-(2,1-Benzisothiazol-3-yl)-1-[2-(7-methyl-4-oxo-5,6,7,8-tetrahydropyrido[4',3':4,5]thieno[2,3-d]pyrimidin-3(4H)-yl)ethyl]piperazin-1-iumchlorid

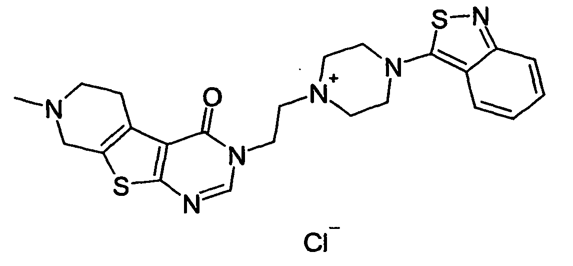

### Beispiel 32:

3-{2-[4-(5-methoxy-1H-indol-3-yl)-3,6-dihydropyridin-1(2H)-yl]ethyl}-7-methyl-5,6,7,8-tetrahydropyrido[4',3':4,5]thieno[2,3-d]pyrimidin-4(3H)-one

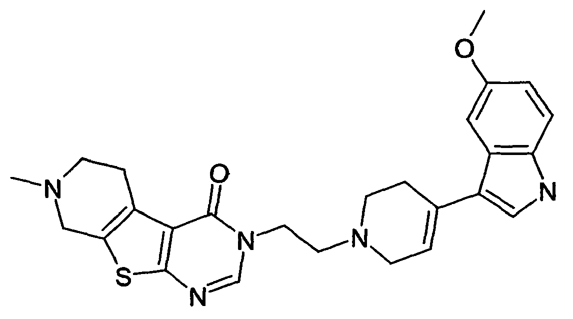

### Beispiel 33:

4-(5-Methoxy-1-methyl-1H-indol-3-yl)-1-[2-(7-methyl-4-oxo-5,6,7,8-tetrahydropyrido[4',3':4,5]thieno[2,3-d]pyrimidin-3(4H)-yl)ethyl]-1,2,3,6-tetrahydropyrimidiniumchlorid

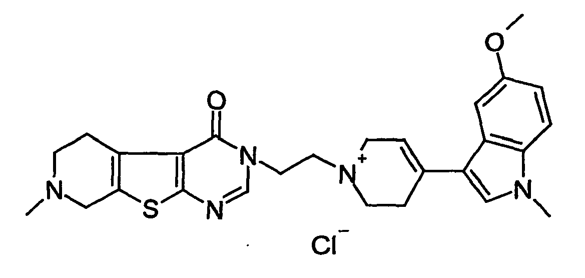

### Beispiel 34:

3-{(1R)-1-methyl-2-[4-(5-methyl-1,2-benzisoxazol-3-yl)piperazin-1-yl]ethyl}-3,5,6,8-tetrahydro-4H-pyrano[4',3':4,5]thieno[2,3-d]pyrimidin-4-on
ESI-MS [M+H⁺] = 466.

### Rezeptorbindungsstudien

### Membranpräparation

HEK293-Zellen, die 5-HT_{1A}- oder 5-HT_{1B}-Rezeptoren exprimierten, wurden in 10 % fötales Rinderserum enthaltendem RPMI 1640 (Life Technologies, Eggenstein, Deutschland) kultiviert. Sämtliche Kulturmedien wurden mit 2 mM L-Glutamin und 400 mg/l Geneticin G418 (Life Technologies) supplementiert. Die Zellen wurden in einer Feuchtatmosphäre mit 6 % CO₂ bei 37 °C angezogen und mit einem 0,0004 % EDTA, 0,02 % EGTA, 2,68 mM KCl, 1,47 mM KH₂PO₄, 6,46 mM Na₂HPO₄ und 136,9 mM NaCl enthaltenden Puffer (pH 7,4) abgelöst. Die eingesammelten Zellen wurden einmal mit kalter Phosphatgepufferter Dulbecco-Kochsalzlösung (PBS) gewaschen, zentrifugiert (200 xg; 10 Minuten) und auf eine Zelldichte von 10⁸-Zellen pro Kryovial eingestellt. Nach erneuter Zentrifugation wurden die Pellets in 1 ml eiskaltem Lösepuffer (5mM Tris/HCl (pH 7,4), 10 % Glycerin) resuspendiert und 30 Minuten bei 4 °C inkubiert. Anschließend wurde die Suspension bei 900 xg 10 Minuten zentrifugiert, die Überstände wurden abgenommen, und die Pellets wurden in flüssigem Stickstoff bis zum Gebrauch aufbewahrt.

### Bindungsexperimente

Zunächst berechnete man die Affinitäten der Radioliganden ((³H)-8-OH-DPAT für 5-HT_{1A}-Rezeptoren; oder (³H)-5-CT für 5-HT_{1B}-Rezeptoren) aus Sättigungskurven, wobei man für jede Testkonzentration Dreifachbestimmungen hinsichtlich der spezifischen und der unspezifischen Bindung durchführte. Jede Probe setzte sich aus 50 µg Zellproteine der jeweiligen Membranpräparation, dem Radioligand mit oder ohne kompetitierender Verbindung (5-HT) bei einem Gesamtvolumen von 0,5 ml zusammen. Es wurde ein Puffer verwendet, der aus 50 mM Tris/HCl (pH 7,4) und 5 mM CaCl₂ bestand. Es wurde 9 Minuten bei 22 °C inkubiert.

Für Ki-Bestimmungen wurden die Testverbindungen in Verdrängungsexperimenten gegen eine festgelegte Konzentration von Radioligand ((0,2 nM (³H)-8-OH-DPAT; oder 0,2 nM (³H)-5-CT) dreimal titriert. Die übrigen Testbedingungen entsprachen den für die Sättigungsexperimente beschriebenen. Für jede experimentelle Reihe wurde die gesamte und die unspezifische Bindung (10 µM 5-HT als Kompetitor für 5-HT_{1A} und 100 µM für 5-HT_{1B}) bestimmt.

Die Inkubation zur Bindung und Verdrängung wurde beendet, indem man die Proben über Glasfaserfilter (GF/B) mit einem Skatron-Zellsammler 7200 (Zinsser, Frankfurt, Deutschland) filtrierte und wusch. Die Probenfilter wurden in 5 ml Scintillator (Ultima Gold XR) enthaltende Scintillationsgläschen überführt und 1 Stunde bewegt, und anschließend in einem Flüssigkeitsscintillationszähler gemessen. Sättigungs- und Kompetitionskurven wurden mit Hilfe eines Computerprogramms berechnet, das auf LIGAND (Mundson, PJ und Reodbard, D; Anal. Biochem. 107, 220 (1980)) basierte.

Folgende Bindungsaffinitäten wurden bestimmt:

| Beispiel | Ki 5HT_{1A} [nM] | Ki 5HT_{1B} [nM] |
|---|---|---|
| 4 | 1,4 | 0,9 |
| 9 | 6,2 | 4,5 |
| 13 | 11,5 | 3,5 |
| 15 | 13,7 | 7,9 |
| 16 | 1,3 | 2,3 |
| 20 | 1,2 | 1,0 |
| 21 | 14,6 | 1,0 |
| 22 | 17,0 | 2,7 |
| 24 | 98,3 | 286,1 |
| 28 | 1,3 | 2,0 |
| 31 | 42,7 | 24,3 |
| 32 | 0,5 | 0,6 |

## Patentansprüche

1. Verbindung der Formel (I) 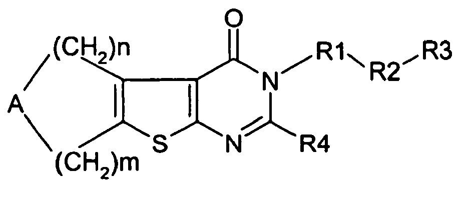 worin
A für O, S, SO, NR5 oder CH₂ steht;
R5 für H, C₁₋₅-Alkyl, Aryl, Aralkyl, Acyl oder Alkoxycarbonyl steht;
R4 für H oder Methyl steht;
n 1 oder 2 bedeutet;
m 1 oder 2 bedeutet;
R1 für C₁₋₈-Alkylen steht;
R2 für eine Gruppe der Formel 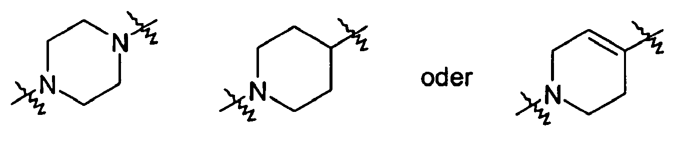 steht;
R3 für 5-gliedriges Heteroaryl steht, das mit einem Aryl- oder Heteroarylrest kondensiert sein kann, wobei das Heteroaryl und gegebenenfalls der kondensierte Aryl- oder Heteroarylrest 1, 2 oder 3 unabhängig voneinander unter C₁₋₅-Alkyl, C₁₋₅-Alkoxy, C₁₋₅-Alkylthio, Halogen, CN, Halogen-C₁₋₅-alkyl, Halogen-C₁₋₅-alkoxy, Hydroxy, -NH₂, -N(R6)₂, -NH(R6), Aryl, Aryloxy, Aralkyl, Aralkyloxy und Heteroaryl ausgewählte Substituenten tragen können, wobei die Substituenten Aryl, Aryloxy, Aralkyl, Aralkyloxy und Heteroaryl 1, 2 oder 3 unabhängig voneinander unter C₁₋₅-Alkyl, C₁₋₅-Alkoxy, C₁₋₅-Alkylthio, Halogen, CN, Halogen-C₁₋₅-alkyl, Halogen-C₁₋₅-alkoxy, Hydroxy, -NH₂, -N(R6)₂ und -NH(R6) ausgewählte Substituenten tragen können; und die Reste
R6 unabhängig voneinander für C₁₋₅-Alkyl stehen,
sowie physiologisch verträgliche Salze davon.

2. Verbindung nach Anspruch 1, **dadurch gekennzeichnet, dass** R3 für 1 H-Indol-3-yl, 1H-Pyrrolo[2,3-b]pyridin-3-yl, 1-Benzofuran-3-yl, 1-Benzothien-3-yl, 1H-Indazol-3-yl, 1H-Benzimidazol-1-yl, 1H-Benzimidazol-2-yl, 1H-Benzotriazol-1-yl, 1,3-Benzoxazol-2-yl, 1,2-Benzisoxazol-3-yl, 1,3-Benzothiazol-2-yl, 1,2-Benzisothiazol-3-yl, Pyrazol-3-yl, 1 H-Tetrazol-5-yl, 1,3-Thiazol-2-yl oder 1,2,4-Thiadiazol-5-yl steht, das 1, 2 oder 3 unabhängig voneinander unter C₁₋₅-Alkyl, C₁₋₅-Alkoxy, Halogen, CN, SCH₃, Trifluormethyl, Hydroxy, -N(C₁₋₅-Alkyl)₂, -NH(C₁₋₅-Alkyl), -NH₂, Aryl-, Aryloxy, Aralkyl, Aralkyloxy und Heteroaryl ausgewählte Substituenten tragen kann, wobei die Substituenten Aryl-, Aryloxy, Aralkyl, Aralkyloxy und Heteroaryl 1, 2 oder 3 unabhängig voneinander unter C₁₋₅-Alkyl, C₁₋₅-Alkoxy, Halogen, CN, SCH₃, Trifluormethyl, Hydroxy, -N(C₁₋₅-Alkyl)₂, -NH(C₁₋₅-Alkyl) oder -NH₂ ausgewählte Substituenten tragen können.

3. Verbindung nach Anspruch 2, **dadurch gekennzeichnet, dass** R3 für einen Rest der Formel 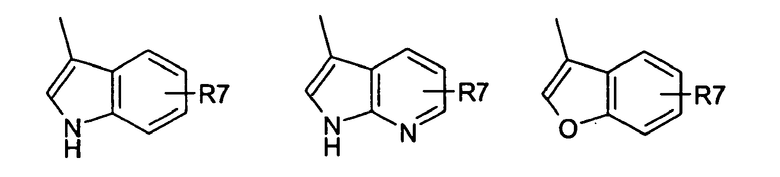 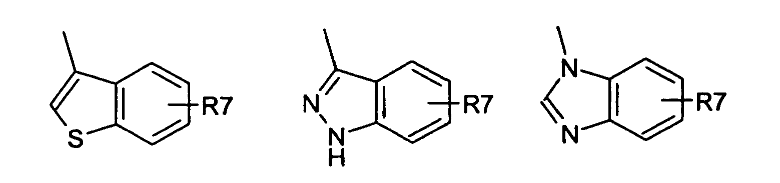 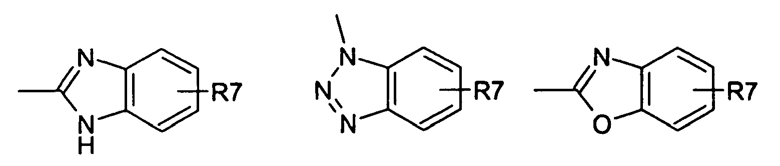 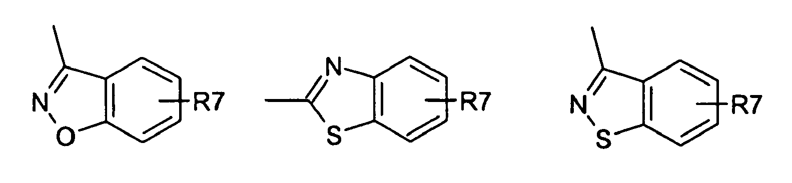 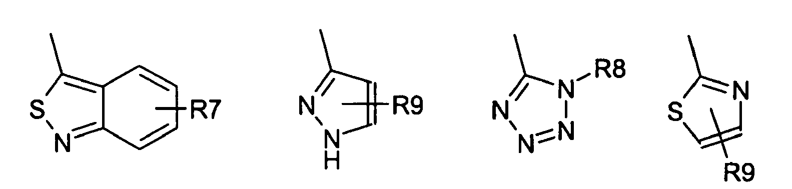 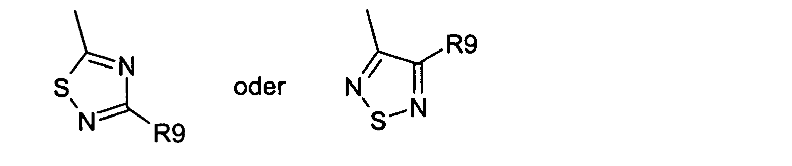 steht, worin
R7 für H, C₁₋₅-Alkyl, C₁₋₅-Alkoxy, C₁₋₅-Alkylthio, Halogen, CN, Halogen-C₁₋₅-alkyl, Halogen-C₁₋₅-alkoxy, Hydroxy, -NH₂, -N(R6)₂ oder -NH(R6) steht; und
R8 für H, C₁₋₅-Alkyl, Aryl, Aralkyl und Heteroaryl steht;
R9 für H, C₁₋₅-Alkyl, C₁₋₅-Alkoxy, C₁₋₅-Alkylthio, Halogen, CN, Halogen-C₁₋₅-alkyl, Halogen-C₁₋₅-alkoxy, Hydroxy, -NH₂, -N(R6)₂, -NH(R6), Aryl, Aryloxy, Aralkyl, Aralkyloxy oder Heteroaryl steht, wobei Aryl, Aryloxy, Aralkyl, Aralkyloxy oder Heteroaryl 1, 2 oder 3 unabhängig voneinander unter C₁₋₅-Alkyl, C₁₋₅-Alkoxy, C₁₋₅-Alkylthio, Halogen, CN, Halogen-C₁₋₅-alkyl, Halogen-C₁₋₅-alkoxy, Hydroxy, -NH₂, -N(R6)₂ und -NH(R6) ausgewählte Substituenten tragen können; und die Reste
R6 die in Anspruch 1 angegebene Bedeutung besitzen.

4. Verbindung nach Anspruch 3, **dadurch gekennzeichnet, dass** R3 für einen Rest der Formel 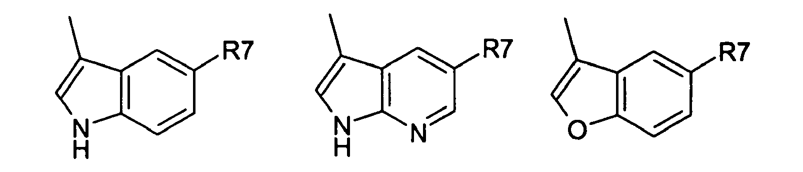 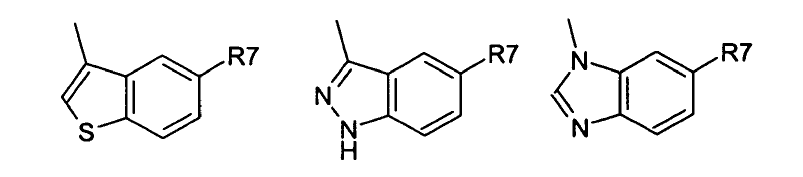 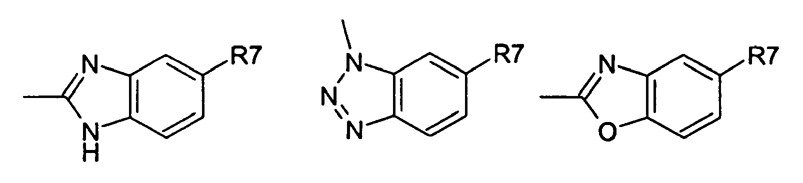 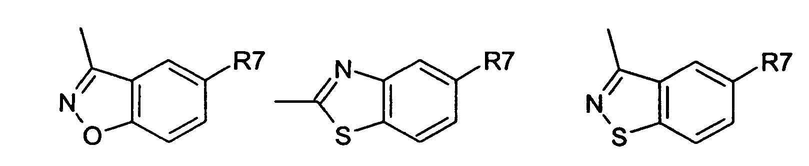 oder 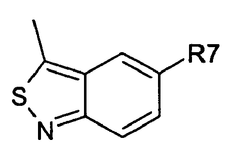 steht, worin R7 wie in Anspruch 3 definiert ist.

5. Verbindung nach Anspruch 3, **dadurch gekennzeichnet, dass** R3 für einen Rest der Formel 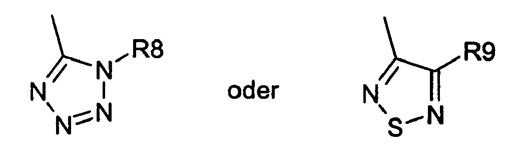 steht, wobei R8 und R9 wie in Anspruch 3 definiert sind.

6. Verbindung nach Anspruch 4, **dadurch gekennzeichnet, dass** R7 für H, C₁₋₅-Alkyl, vorzugsweise Methyl, Halogen, vorzugsweise Chlor, oder Halogen-C₁₋₅-Alkyl, vorzugsweise Trifluormethyl, steht.

7. Verbindung nach Anspruch 5, **dadurch gekennzeichnet, dass** R8 für C₁₋₅-Alkyl, vorzugsweise Methyl, Ehyl oder Isopropyl oder Aryl, vorzugsweise Phenyl, steht.

8. Verbindung nach Anspruch 5, **dadurch gekennzeichnet, dass** R9 für C₁₋₅-Alkoxy, vorzugsweise Methoxy, Ethoxy oder Isopropoxy, Aryl, vorzugsweise Phenyl, das substituiert sein kann, z.B. mit Chlor, oder Heteroaryl, z.B. 2-Thienyl, steht.

9. Verbindung nach einem der Ansprüche 1 bis 8, **dadurch gekennzeichnet, dass** A für O, S oder NR5 steht, wobei R5 wie in Anspruch 1 definiert und vorzugsweise H oder Methyl ist.

10. Verbindung nach einem der Ansprüche 1 bis 8, **dadurch gekennzeichnet, dass** R4 Wasserstoff ist.

11. Verbindung nach einem der Ansprüche 1 bis 8, **dadurch gekennzeichnet, dass** n = 2 und m = 1 oder n = 1 und m = 2 ist.

12. Verbindung nach einem der Ansprüche 1 bis 8, **dadurch gekennzeichnet, dass** R1 Eth-1,2-ylen, Prop-1,3-ylen, Prop-1,2-ylen, 2-Methyl-prop-1,3-ylen, But-1,2-ylen oder But-1,3-ylen ist.

13. Verbindung nach einem der Ansprüche 1 bis 8, **dadurch gekennzeichnet, dass**
R4 Wasserstoff ist;
n, m 2, 1 oder 1, 2 sind;
R1 Eth-1,2-ylen, Prop-1,3-ylen, Prop-1,2-ylen, 2-Methyl-prop-1,3-ylen, But-1,2-ylen oder But-1,3-ylen ist;
R2 für eine Gruppe der Formel 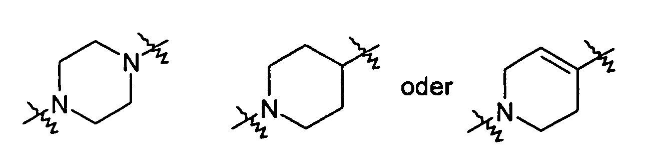 steht; und
R3 wie in einem der Ansprüche 1 bis 13 definiert ist;

14. Verbindung nach Anspruch 13, nämlich
3-substituierte 5,6,7,8-Tetrahydro-pyrido[4',3':4,5]thieno[2,3-d]pyrimidin-4(3H)-on-Derivate;
3-substituierte 3,5,6,8-Tetrahydro-4H-pyrano[4',3':4,5]thieno[2,3-d]pyrimidin-4-on-Derivate, oder
3-substituierte 3,5,6,8-Tetrahydro-4H-thiopyrano[4',3';4,5]thieno[2,3-d]pyrimidin-4-on- Derivate.

15. Verfahren zur Herstellung einer Verbindung nach einem der Ansprüche 1 bis 14,
a) durch Umsetzung einer Verbindung der Formel (II) 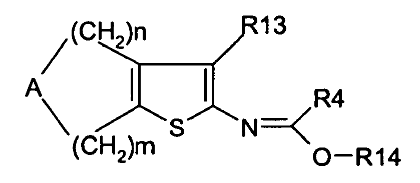
worin A, n, m und R4 eine der in Anspruch 1 angegebenen Bedeutung besitzen; R13 für CN oder C₁₋₃-Alkyl-O-CO- steht und R14 für C₁₋₃-Alkyl steht,
mit einem primären Amin der Formel (III) 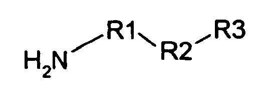 worin R1, R2 und R3 eine der in Anspruch 1 angegebenen Bedeutung besitzen, sowie Gewinnung und gegebenenfalls Überführung der resultierenden Verbindung in ein physiologisch verträgliches Salze davon, oder
b1) durch Umsetzung einer Verbindung der Formel (II) 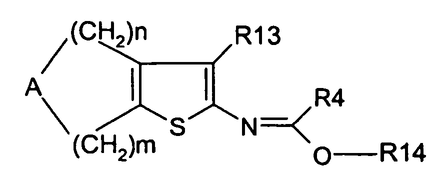 worin A, n, m und R4 eine der in Anspruch 1 angegebenen Bedeutung besitzen; R13 für CN oder C₁₋₃-Alkyl-O-CO- steht und R14 für C₁₋₃-Alkyl steht,
mit einem primären Amin der Formel (IV)
H₂N-R1-OH
worin R1 eine der in Anspruch 1 angegebenen Bedeutung besitzt;
b2) Umsetzung der resultierenden Verbindung der Formel (V) 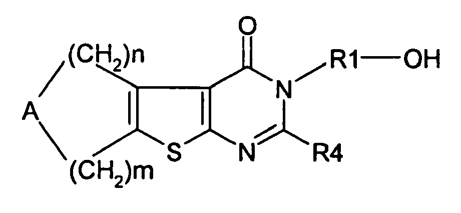 worin A, n, m, R4 und R1 eine der in Anspruch 1 angegebenen Bedeutung besitzen,
mit einem Halogenierungsmittel wie Thionylchlorid; und
b3) Umsetzung der resultierenden Verbindung der Formel (VI) 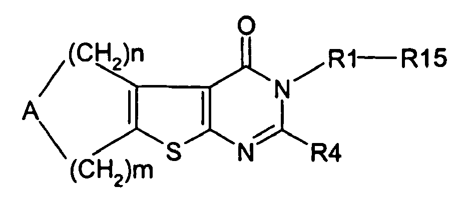 worin A, n, m, R4 und R1 eine der in Anspruch 1 angegebenen Bedeutung besitzen und R15 für Halogen steht,
mit einem sekundären Amin der Formel (VII)
H-R2-R3
worin R2 und R3 eine der in Anspruch 1 angegebenen Bedeutung besitzen,
sowie Gewinnung und gegebenenfalls Überführung der resultierenden Verbindung in ein physiologisch verträgliches Salze davon.

16. Verbindung nach einem der Ansprüche 1 bis 14 zur therapeutischen Anwendung.

17. Pharmazeutisches Mittel, enthaltend wenigstens eine Verbindung nach einem der Ansprüche 1 bis 14 und physiologisch akzeptable Hilfsmittel.

18. Verwendung einer Verbindung nach einem der Ansprüche 1 bis 14 zur Herstellung eines Mittels zur Behandlung von Erkrankungen des zentralen Nervensystems.

19. Verwendung nach 18, **dadurch gekennzeichnet, dass** die Erkrankung des zentralen Nervensystems eine neuropsychiatrische Erkrankung ist.

20. Verwendung nach Anspruch 19, **dadurch gekennzeichnet, dass** die neuropsychiatrische Erkrankung eine Depression ist.

## Claims

1. A compound of the formula (I) 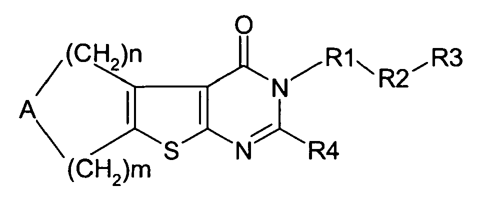 in which
A is O, S, SO, NR5 or CH₂;
R5 is H, C₁₋₅-alkyl, aryl, aralkyl, acyl or alkoxycarbonyl;
R4 is H or methyl;
n is 1 or 2;
m is 1 or 2;
R1 is C₁₋₈-alkylene;
R2 is a group of the formula 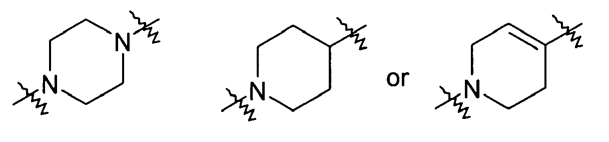
R3 is 5-membered heteroaryl which may be fused to an aryl or heteroaryl radical, where the heteroaryl and, if appropriate, the fused aryl or heteroaryl radical may have 1, 2 or 3 substituents selected independently of one another from C₁₋₅-alkyl, C₁₋₅-alkoxy, C₁₋₅-alkylthio, halogen, CN, halo-C₁₋₅-alkyl, halo-C₁₋₅-alkoxy, hydroxy, -NH₂, -N(R6)₂, -NH(R6), aryl, aryloxy, aralkyl, aralkyloxy and heteroaryl, where the substituents aryl, aryloxy, aralkyl, aralkyloxy and heteroaryl may have 1, 2 or 3 substituents selected independently of one another from C₁₋₅-alkyl, C₁₋₅-alkoxy, C₁₋₅-alkylthio, halogen, CN, halo-C₁₋₅-alkyl, halo-C₁₋₅-alkoxy, hydroxy, -NH₂, -N(R6)₂ and -NH(R6); and the radicals
R6 are independently of one another C₁₋₅-alkyl,
and physiologically tolerated salts thereof.

2. The compound according to claim 1, wherein R3 is 1 H-indol-3-yl, 1 H-pyrrolo[2,3-b]pyridin-3-yl, 1-benzofuran-3-yl, 1-benzothien-3-yl, 1H-indazol-3-yl, 1 H-benzimidazol-1-yl, 1H-benzimidazol-2-yl, 1H-benzotriazol-1-yl, 1,3-benzoxazol-2-yl, 1,2-benzisoxazol-3-yl, 1,3-benzothiazol-2-yl, 1,2-benzisothiazol-3-yl, pyrazol-3-yl, 1 H-tetrazol-5-yl, 1,3-thiazol-2-yl or 1,2,4-thiadiazol-5-yl, which may have 1, 2 or 3 substituents selected independently of one another from C₁₋₅-alkyl, C₁₋₅-alkoxy, halogen, CN, SCH₃, trifluoromethyl, hydroxy, -N(C₁₋₅-alkyl)₂, -NH(C₁₋₅-alkyl), -NH₂, aryl, aryloxy, aralkyl, aralkyloxy and heteroaryl, where the substituents aryl, aryloxy, aralkyl, aralkyloxy and heteroaryl may have 1, 2 or 3 substituents selected independently of one another from C₁₋₅-alkyl, C₁₋₅-alkoxy, halogen, CN, SCH₃, trifluoromethyl, hydroxy, -N(C₁₋₅-alkyl)₂, -NH(C₁₋₅-alkyl) or -NH₂.

3. The compound according to claim 2, wherein R3 is a radical of the formula 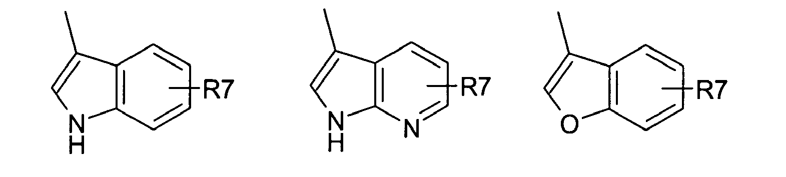 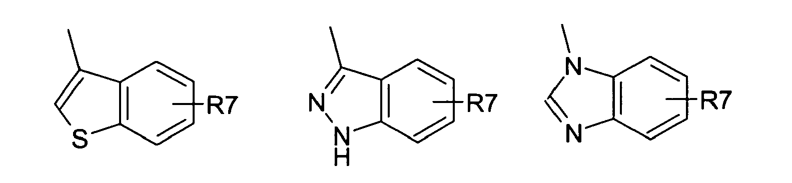 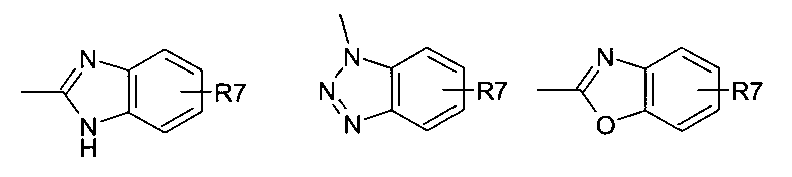 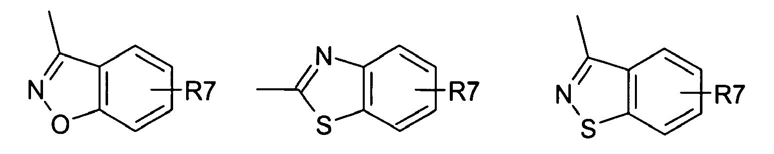 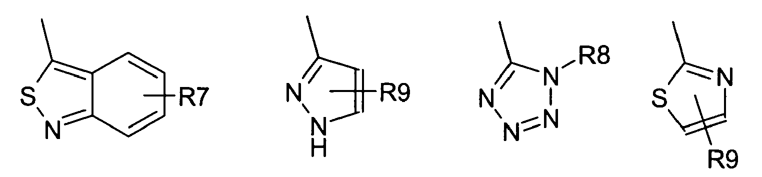 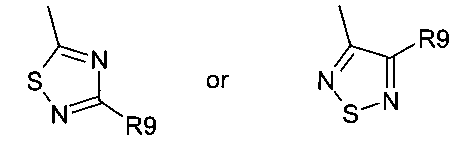 in which
R7 is H, C₁₋₅-alkyl, C₁₋₅-alkoxy, C₁₋₅-alkylthio, halogen, CN, halo-C₁₋₅-alkyl, halo-C₁₋₅-alkoxy, hydroxy, -NH₂, -N(R6)₂ or -NH(R6); and
R8 is H, C₁₋₅-alkyl, aryl, aralkyl and heteroaryl;
R9 is H, C₁₋₅-alkyl, C₁₋₅-alkoxy, C₁₋₅-alkylthio, halogen, CN, halo-C₁₋₅-alkyl, halo-C₁₋₅-alkoxy, hydroxy, -NH₂, -N(R6)₂, -NH(R6), aryl, aryloxy, aralkyl, aralkyloxy or heteroaryl, where aryl, aryloxy, aralkyl, aralkyloxy or heteroaryl may have 1, 2 or 3 substituents selected independently of one another from C₁₋₅-alkyl, C₁₋₅-alkoxy, C₁₋₅-alkylthio, halogen, CN, halo-C₁₋₅-alkyl, halo-C₁₋₅-alkoxy, hydroxy, -NH₂, -N(R6)₂ and -NH(R6); and the radicals
R6 have the meaning indicated in claim 1.

4. The compound according to claim 3, wherein R3 is a radical of the formula 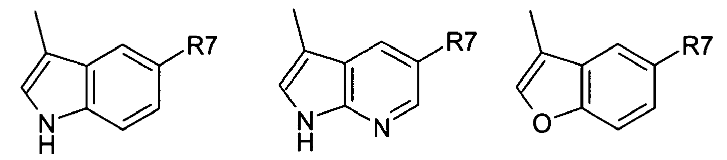 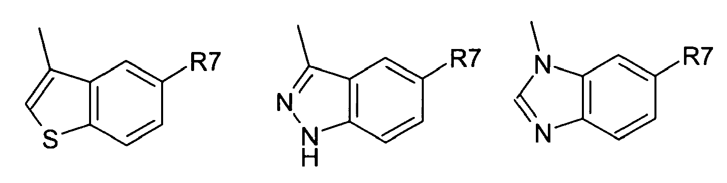 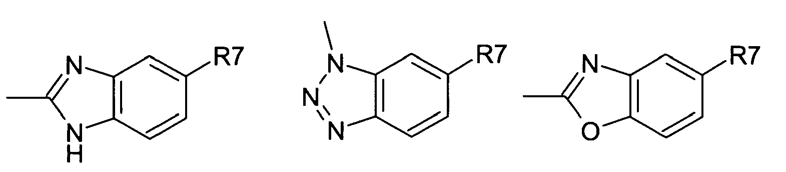 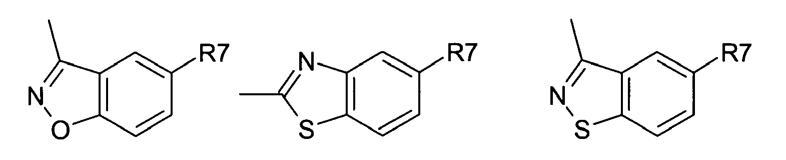 or 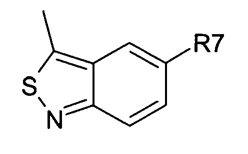 in which R7 is as defined in claim 3.

5. The compound according to claim 3, wherein R3 is a radical of the formula 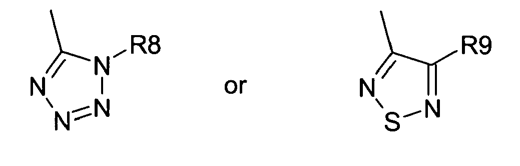 where R8 and R9 are as defined in claim 3.

6. The compound according to claim 4, wherein R7 is H, C₁₋₅-alkyl, preferably methyl, halogen, preferably chlorine, or halo-C₁₋₅-alkyl, preferably trifluoromethyl.

7. The compound according to claim 5, wherein R8 is C₁₋₅-alkyl, preferably methyl, ethyl or isopropyl or aryl, preferably phenyl.

8. The compound according to claim 5, wherein R9 is C₁₋₅-alkoxy, preferably methoxy, ethoxy or isopropoxy, aryl, preferably phenyl which may be substituted, e.g. by chlorine, or heteroaryl, e.g. 2-thienyl.

9. The compound according to any of claims 1 to 8, wherein A is O, S or NR5, where R5 is as defined in claim 1 and is preferably H or methyl.

10. The compound according to any of claims 1 to 8, wherein R4 is hydrogen.

11. The compound according to any of claims 1 to 8, wherein n is 2 and m is 1 or n is 1 and m is 2.

12. The compound according to any of claims 1 to 8, wherein R1 is eth-1,2-ylene, prop-1,3-ylene, prop-1,2-ylene, 2-methyl-prop-1,3-ylene, but-1,2-ylene or but-1,3-ylene.

13. The compound according to any of claims 1 to 8, wherein
R4 is hydrogen;
n, m are 2, 1 or 1, 2;
R1 is eth-1,2-ylene, prop-1,3-ylene, prop-1,2-ylene, 2-methylprop-1,3-ylene, but-1,2-ylene or but-1,3-ylene;
R2 is a group of the formula 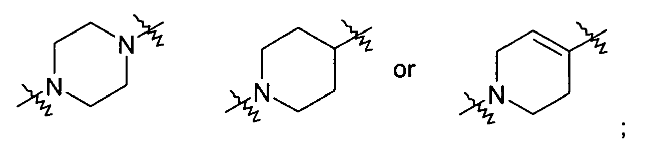 and
R3 is as defined in any of claims 1 to 13;

14. The compound according to claim 13, namely
3-substituted 5,6,7,8-tetrahydropyrido[4',3':4,5]thieno[2,3-d]pyrimidin-4(3H)-one derivatives;
3-substituted 3,5,6,8-tetrahydro-4H-pyrano[4',3':4,5]thieno[2,3-d]pyrimidin-4-one derivatives, or
3-substituted 3,5,6,8-tetrahydro-4H-thiopyrano[4',3';4,5]thieno[2,3-d]pyrimidin-4-one derivatives.

15. A process for preparing a compound according to any of claims 1 to 14
a) by reacting a compound of the formula (II) 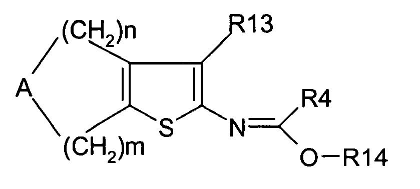
in which A, n, m and R4 have one of the meaning indicated in claim 1; R13 is CN or C₁₋₃-alkyl-O-CO-, and R14 is C₁₋₃-alkyl,
with a primary amine of the formula (III) 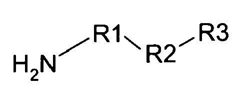 in which R1, R2 and R3 have one of the meaning indicated in claim 1, and isolating and, if appropriate, converting the resulting compound into a physiologically tolerated salt thereof, or
b1) by reacting a compound of the formula (II) 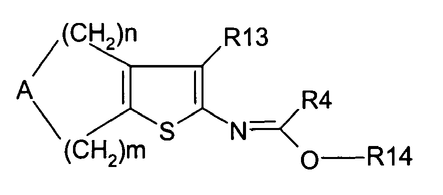 in which A, n, m and R4 have one of the meaning indicated in claim 1; R13 is CN or C₁₋₃-alkyl-O-CO-, and R14 is C₁₋₃-alkyl,
with a primary amine of the formula (IV)
H₂N-R1-OH
in which R1 has one of the meaning indicated in claim 1;
b2) reacting the resulting compound of the formula (V) 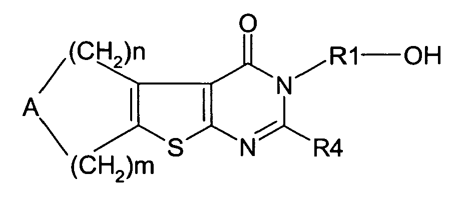 in which A, n, m, R4 and R1 have one of the meaning indicated in claim 1,
with a halogenating agent such as thionyl chloride; and
b3) reacting the resulting compound of the formula (VI) 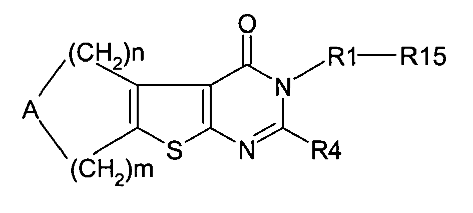 in which A, n, m, R4 and R1 have one of the meaning indicated in claim 1, and R15 is halogen,
with a secondary amine of the formula (VII)
H-R2-R3
in which R2 and R3 have one of the meaning indicated in claim 1, and isolating and, if appropriate, converting the resulting compound into a physiologically tolerated salt thereof.

16. The compound according to any of claims 1 to 14 for therapeutic use.

17. A pharmaceutical composition comprising at least one compound according to any of claims 1 to 14 and physiologically acceptable aids.

18. The use of a compound according to any of claims 1 to 14 for producing a composition for the treatment of disorders of the central nervous system.

19. The use according to 18, wherein the disorder of the central nervous system is a neuropsychiatric disorder.

20. The use according to claim 19, wherein the neuropsychiatric disorder is a depression.

## Revendications

1. Composé de formule (I) 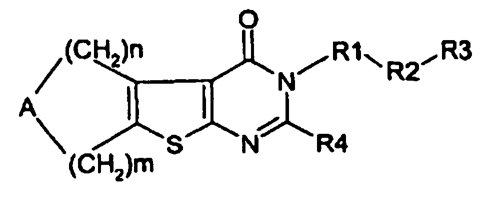 dans laquelle
A représente O, S, SO, NR5 ou CH₂;
R5 représente H, alkyle en C₁-C₅, aryle, aralkyle, acyle ou alcoxycarbonyle;
R4 représente H ou méthyle;
n signifie 1 ou 2;
m signifie 1 ou 2;
R1 représente un alkylène en C₁-C₈;
R2 représente un groupe de formule 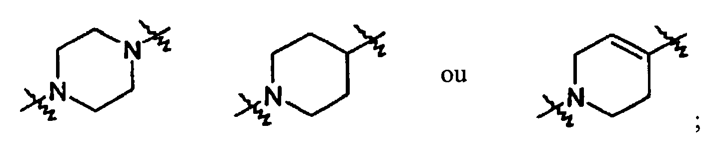
R3 représente un hétéroaryle de 5 chaînons qui peut être condensé avec un reste aryle ou hétéroaryle, l'hétéroaryle et éventuellement le reste aryle ou hétéroaryle condensé pouvant porter 1, 2 ou 3 substituants choisis indépendamment les uns des autres parmi alkyle en C₁-C₅, alcoxy en C₁-C₅, alkylthio en C₁-C₅, halogéno, CN, halogénoalkyle en C₁-C₅, halogénoalcoxy en C₁-C₅, hydroxy, -NH₂, -N(R6)₂, -NH(R6), aryle, aryloxy, aralkyle, aralkyloxy et hétéroaryle, les substituants aryle, aryloxy, aralkyle, aralkyloxy et hétéroaryle pouvant porter 1, 2 ou 3 substituants choisis indépendamment les uns des autres parmi alkyle en C₁-C₅, alcoxy en C₁-C₅, alkylthio en C₁-C₅, halogéno, CN, halogénoalkyle en C₁-C₅, halogénoalcoxy en C₁-C₅, hydroxy, -NH₂, -N(R6)₂ et -NH(R6); et les restes
R6 représentent indépendamment l'un de l'autre un alkyle en C₁-C₅,
et ses sels physiologiquement acceptables.

2. Composé selon la revendication 1, **caractérisé en ce que** R3 représente un reste 1H-indol-3-yle, 1H-pyrrolo[2,3-b]pyridin-3-yle, 1-benzofuran-3-yle, 1-benzothién-3-yle, 1H-indazol-3-yle, 1H-benzimidazol-1-yle, 1H-benzimidazol-2-yle, 1H-benzotriazol-1-yle, 1,3-benzoxazol-2-yle, 1,2-benzisoxazol-3-yle, 1,3-benzothiazol-2-yle, 1,2-benzisothiazol-3-yle, pyrazol-3-yle, 1H-tétrazol-5-yle, 1,3-thiazol-2-yle ou 1,2,4-thiadiazol-5-yle, qui peut porter 1, 2 ou 3 substituants choisis indépendamment les uns des autres parmi alkyle en C₁-C₅, alcoxy en C₁-C₅, halogéno, CN, SCH₃, trifluorométhyle, hydroxy, -N(alkyle en C₁-C₅)₂, -NH(alkyle en C₁-C₅), -NH₂, aryle, aryloxy, aralkyle, aralkyloxy et hétéroaryle, les substituants aryle, aryloxy, aralkyle, aralkyloxy et hétéroaryle pouvant porter 1, 2 ou 3 substituants choisis indépendamment les uns des autres parmi alkyle en C₁-C₅, alcoxy en C₁-C₅, halogéno, CN, SCH₃, trifluorométhyle, hydroxy, -N(alkyle en C₁-C₅)₂, -NH(alkyle en C₁-C₅) ou -NH₂.

3. Composé selon la revendication 2, **caractérisé en ce que** R3 représente un reste de formule 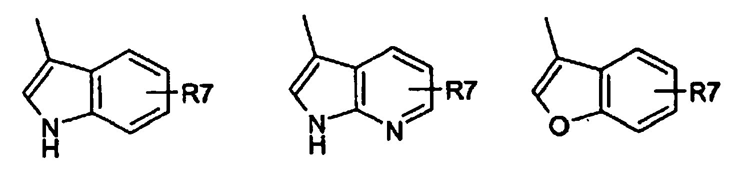 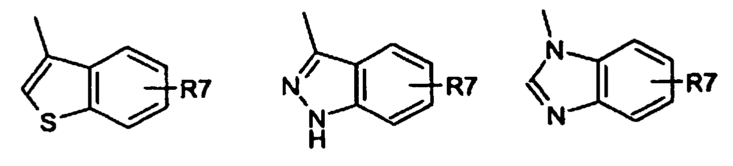 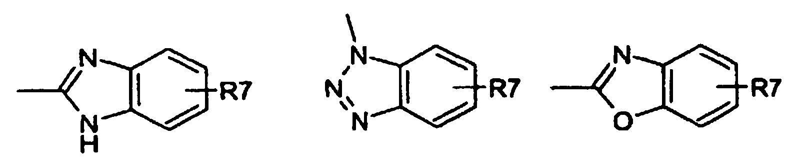 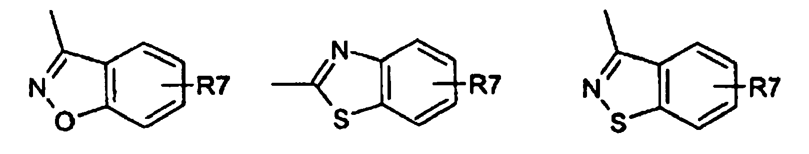 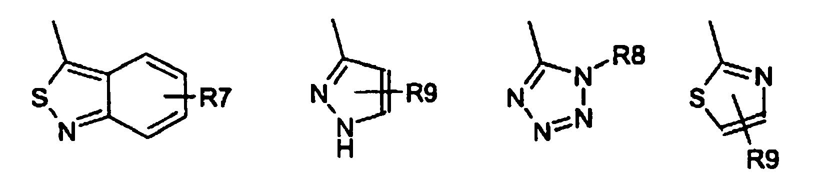 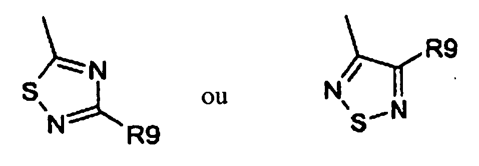 où
R7 représente H, alkyle en C₁-C₅, alcoxy en C₁-C₅, alkylthio en C₁-C₅, halogéno, CN, halogénoalkyle en C₁-C₅, halogénoalcoxy en C₁-C₅, hydroxy, -NH₂, -N(R6)₂ ou -NH(R6); et
R8 représente H, alkyle en C₁-C₅, aryle, aralkyle et hétéroaryle;
R9 représente H, alkyle en C₁-C₅, alcoxy en C₁-C₅, alkylthio en C₁-C₅, halogéno, CN, halogénoalkyle en C₁-C₅, halogénoalcoxy en C₁-C₅, hydroxy, -NH₂, -N(R6)₂, -NH(R6), aryle, aryloxy, aralkyle, aralkyloxy ou hétéroaryle, les restes aryle, aryloxy, aralkyle, aralkyloxy ou hétéroaryle pouvant porter 1, 2 ou 3 substituants choisis indépendamment les uns des autres parmi alkyle en C₁-C₅, alcoxy en C₁-C₅, alkylthio en C₁-C₅, halogéno, CN, halogénoalkyle en C₁-C₅, halogénoalcoxy en C₁-C₅, hydroxy, -NH₂, -N(R6)₂ et -NH(R6); et les restes
R6 ont la signification donnée dans la revendication 1.

4. Composé selon la revendication 3, **caractérisé en ce que** R3 représente un reste de formule 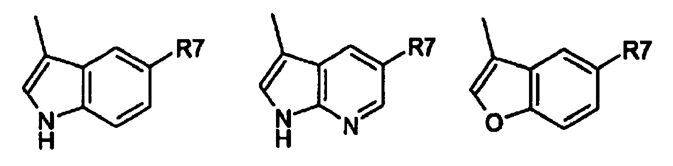 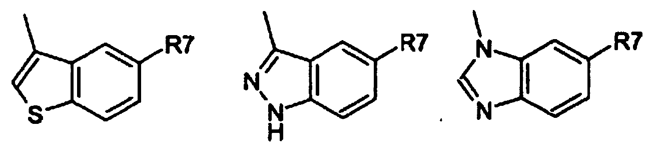 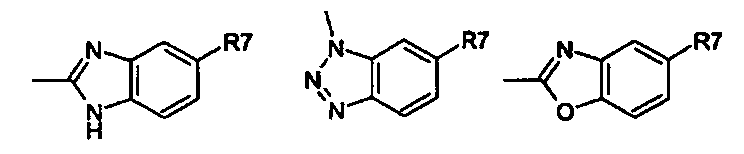 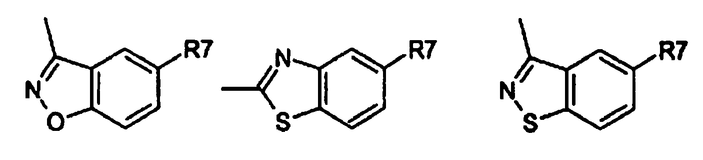 ou 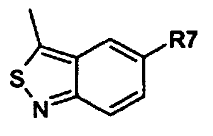 où R7 a la définition donnée dans la revendication 3.

5. Composé selon la revendication 3, **caractérisé en ce que** R3 représente un reste de formule 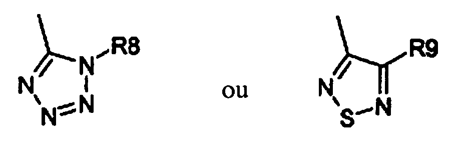 où R8 et R9 ont la signification donnée dans la revendication 3.

6. Composé selon la revendication 4, **caractérisé en ce que** R7 représente H, alkyle en C₁-C₅, de préférence méthyle, halogéno, de préférence chloro, ou halogénoalkyle en C₁-C₅, de préférence trifluorométhyle.

7. Composé selon la revendication 5, **caractérisé en ce que** R8 représente alkyle en C₁-C₅, de préférence méthyle, éthyle ou isopropyle, ou aryle, de préférence phényle.

8. Composé selon la revendication 5, **caractérisé en ce que** R9 représente alcoxy en C₁-C₅, de préférence méthoxy, éthoxy ou isopropoxy, aryle, de préférence phényle, qui peut être substitué, par exemple par chloro, ou hétéroaryle, par exemple 2-thiényle.

9. Composé selon l'une des revendications 1 à 8, **caractérisé en ce que** A est O, S ou NR5, où R5 a la définition donnée dans la revendication 1 et est de préférence H ou méthyle.

10. Composé selon l'une des revendications 1 à 8, **caractérisé en ce que** R4 est l'hydrogène.

11. Composé selon l'une des revendications 1 à 8, **caractérisé en ce que** n = 2 et m = 1 ou n = 1 et m = 2.

12. Composé selon l'une des revendications 1 à 8, **caractérisé en ce que** R1 est éth-1,2-ylène, prop-1,3-ylène, prop-1,2-ylène, 2-méthylprop-1,3-ylène, but-1,2-ylène ou but-1,3-ylène.

13. Composé selon l'une des revendications 1 à 8, **caractérisé en ce que** R4 est l'hydrogène;
n, m sont 2, 1 ou 1, 2;
R1 est éth-1,2-ylène, prop-1,3-ylène, prop-1,2-ylène, 2-méthylprop-1,3-ylène, but-1,2-ylène ou but-1,3-ylène;
R2 représente un groupe de formule 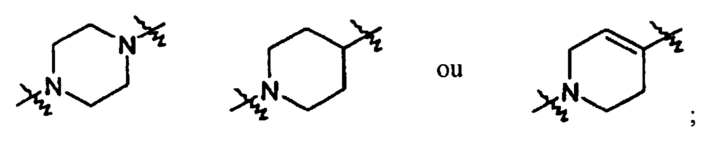 et
R3 a la définition donnée dans l'une des revendications 1 à 13.

14. Composé selon la revendication 13, à savoir:
un dérivé de 5,6,7,8-tétrahydropyrido[4',3':4,5]thiéno[2,3-d]pyrimidin-4(3H)-one substituée en 3 ;
un dérivé de 3,5,6,8-tétrahydro-4H-pyrano[4',3':4,5]thiéno[2,3-d]pyrimidin-4-one substituée en 3, ou
un dérivé de 3,5,6,8-tétrahydro-4H-thiopyrano[4',3':4,5]thiéno[2,3-d]pyrimidin-4-one substituée en 3.

15. Procédé de préparation d'un composé selon l'une des revendications 1 à 14,
a) par réaction d'un composé de formule (II) 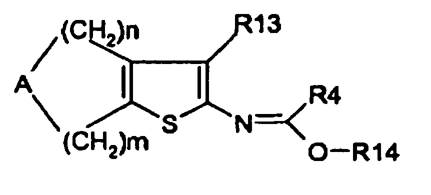
dans laquelle A, n, m et R4 ont l'une des significations données dans la revendication 1; R13 représente CN ou (alkyl en C₁-C₃)-O-CO- et R14 représente un alkyle en C₁-C₃,
avec une amine primaire de formule (III) 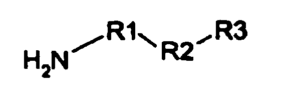 dans laquelle R1, R2 et R3 ont l'une des significations données dans la revendication 1,
et récupération et éventuellement transformation du composé obtenu en un de ses sels physiologiquement acceptables, ou
b1) par réaction d'un composé de formule (II) 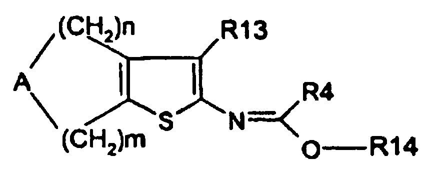 dans laquelle A, n, m et R4 ont l'une des significations données dans la revendication 1; R13 représente CN ou (alkyl en C₁-C₃)-O-CO- et R14 représente un alkyle en C₁-C₃,
avec une amine primaire de formule (IV)
**H₂N-R1-OH**
dans laquelle R1 a l'une des significations données dans la revendication 1; b2) réaction du composé obtenu de formule (V) 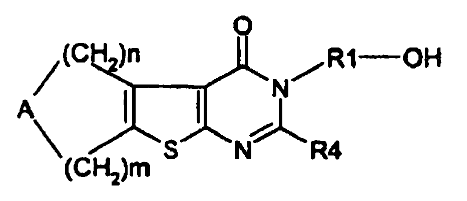 dans laquelle A, n, m, R4 et R1 ont l'une des significations données dans la revendication 1;
avec un agent d'halogénation comme le chlorure de thionyle; et
b3) réaction du composé obtenu de formule (VI) 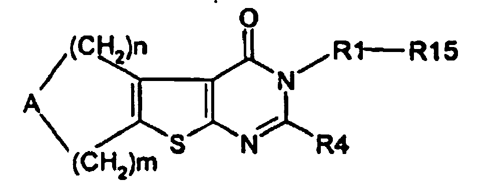 dans laquelle A, n, m, R4 et R1 ont l'une des significations données dans la revendication 1 et R15 représente un halogène,
avec une amine secondaire de formule (VII)
H-R2-R3
dans laquelle R2 et R3 ont l'une des significations données dans la revendication 1, et récupération et éventuellement transformation du composé obtenu en un de ses sels physiologiquement acceptables.

16. Composé selon l'une des revendications 1 à 14 pour une application thérapeutique.

17. Composition pharmaceutique, contenant au moins un composé selon l'une des revendications 1 à 14 et des agents auxiliaires physiologiquement acceptables.

18. Utilisation d'un composé selon l'une des revendications 1 à 14 pour la préparation d'une composition destinée au traitement de maladies du système nerveux central.

19. Utilisation selon la revendication 18, **caractérisée en ce que** la maladie du système nerveux central est une maladie neuropsychiatrique.

20. Utilisation selon la revendication 19, **caractérisée en ce que** la maladie neuropsychiatrique est une dépression.
